# EUROPEAN PATENT APPLICATION

(11) **EP 4 737 392 A1**
(43) Date of publication of application: **06.05.2026**
(21) Application number: 24877276.6
(22) Date of filing: 11.10.2024
(51) Int. Cl.: C01B 32/80, C07B 61/00, C07C 68/02, C07C 69/96, C07C 249/02, C07C 263/10, C07D 263/44

(54) **METHOD FOR PRODUCING CARBONYL HALIDE**

(30) Priority: 12.10.2023 JP 2023176533
(71) Applicant: National University Corporation Kobe University, Kobe-shi, Hyogo 657-8501 (JP)
(72) Inventor: TSUDA, Akihiko, Kobe-shi, Hyogo 657-8501 (JP)
(74) Representative: Müller-Boré & Partner Patentanwälte PartG mbB
(86) International application number: PCT/JP2024/036434
(87) International publication number: WO 2025/079682

(57) **Abstract**

The objective of the present invention is to provide a method for producing a carbonyl halide safely at a low cost and a low environmental load. The method for producing a carbonyl halide according to the present invention is characterized in comprising the step of irradiating a light to a mixed gas comprising methane, a halogen molecule gas and oxygen.

## Description

### TECHNICAL FIELD

The present invention relates to a method for safely producing a carbonyl halide at a lower cost.

### BACKGROUND ART

A carbonyl halide such as carbonyl chloride is very important as a synthetic intermediate for various compounds. For example, a carbonate derivative is generally produced from carbonyl chloride and a nucleophilic functional group-containing compound.

Carbonyl chloride is however very toxic. For example, carbonyl chloride is easily reacted with water to generate hydrogen chloride and has a history of use as a poisonous gas. In general, carbonyl chloride is produced by a high-heat-generating gas phase reaction of an anhydrous chlorine gas with high purity carbon monoxide in the presence of an activated carbon catalyst (Patent document 1 or the like). Carbon monoxide used in this reaction is also toxic. The basic process to produce carbonyl chloride has not changed much since the 1920s. Such a process to produce carbonyl chloride requires expensive large-scale facilities. An extensive safety assurance is essential in plant design due to the high toxicity of carbonyl chloride and leads to increased production costs. In addition, a process to produce carbonyl chloride in a large scale may possibly cause many environmental problems. Further, carbonyl chloride may be also produced by decomposing triphosgene [bis(trichloromethyl) carbonate] or diphosgene (trichloromethyl chloroformate) with a base such as triethylamine. But it is known that triphosgene and diphosgene are expensive reagents, may be potentially decomposed into carbonyl chloride by some physical or chemical stimuli, and are known to be highly toxic themselves.

The inventor of the present invention has developed the method for producing a halogen and/or a carbonyl halide by irradiating light to a halogenated hydrocarbon in the presence of oxygen (Patent document 2). The method is safe, since the carbonyl halide produced by the method can be immediately reacted with a coexistent amine compound or alcohol compound to be reacted.

The carbonyl halide which has not been used for the reaction can be collected not to be leaked outside by using a trap. For example, the inventor also has developed the method for producing a halogenated carboxylate ester by irradiating light to a mixture containing a halogenated hydrocarbon and an alcohol in the presence of oxygen (Patent document 3). The inventor also has developed the method for producing a carbonate derivative by irradiating light to a composition containing a halogenated hydrocarbon, a nucleophilic functional group-containing compound and a base in the presence of oxygen (Patent document 4 and Patent document 5).

### PRIOR ART DOCUMENT

### PATENT DOCUMENT

Patent document 1: JP H9-59012 A
Patent document 2: JP 2013-181028 A
Patent document 3: WO 2015/156245
Patent document 4: WO 2018/211952
Patent document 5: WO 2018/211953

### DISCLOSURE OF THE INVENTION

### PROBLEMS TO BE SOLVED BY THE INVENTION

The inventor of the present invention has developed the method for producing a carbonyl halide by irradiating light to a halogenated hydrocarbon in the presence of oxygen as described above. The halogenated hydrocarbon may be once used and reused. But since many reusable halogenated hydrocarbons have been once used as a solvent and is liquid under atmospheric temperature and atmospheric pressure, the halogenated hydrocarbon has to be easily purified and the energy to gasify the halogenated hydrocarbon is required for the reaction in a gas phase. In addition, a halogenated hydrocarbon is relatively expensive. Methane is inexpensive and is required to be actively consumed, since the global warming potential of methane as a greenhouse gas is about 25 times as that of carbon dioxide.

Thus, the objective of the present invention is to provide a method for producing a carbonyl halide safely at a low cost and a low environmental load.

### MEANS FOR SOLVING THE PROBLEMS

The inventor of the present invention repeated intensive studies in order to solve the above-described problems. As a result, the inventor completed the present invention by finding that the reaction of methane in the presence of oxygen may possibly cause explosion but a carbonyl halide can be safely produced by irradiating light to a mixed gas containing methane, a halogen molecule and oxygen.

The present invention is hereinafter described.

[1] A method for producing a carbonyl halide, the method comprising the step of:
   irradiating a light to a mixed gas comprising methane, a halogen molecule gas and oxygen.
[2] The method according to the above [1], wherein the carbonyl halide is carbonyl chloride, and the halogen molecule gas is a chlorine gas.
[3] The method according to the above [1] or [2], wherein the light has a peak wavelength of 360 nm or more and 830 nm or less.
[4] The method according to any one of the above [1] to [3], wherein the light is irradiated to the mixed gas at an atmospheric temperature.
[5] The method according to any one of the above [1] to [4], wherein the shortest distance between a light source to irradiate the light and the mixed gas is 1 m or less.
[6] The method according to any one of the above [1] to [5], further comprising the step of producing a sodium halide aqueous solution by neutralizing a hydrogen halide with a basic sodium salt, wherein the hydrogen halide is produced as a side product by irradiating the light to the mixed gas.
[7] The method according to any one of the above [1] to [6], further comprising the step of electrolyzing the sodium halide aqueous solution to produce the halogen molecule and sodium hydroxide.
[8] A method for producing a carbonate compound, the method comprising the steps of:
   producing a carbonyl halide by the method according to any one of the above [1] to [7], and
   reacting an alcohol compound and the carbonyl halide.
[9] A method for producing a halogenated formate ester compound, the method comprising the steps of:
   producing a carbonyl halide by the method according to any one of the above [1] to [7], and
   reacting an alcohol compound and the carbonyl halide.
[10] A method for producing an isocyanate compound, the method comprising the steps of:
   producing a carbonyl halide by the method according to any one of the above [1] to [7], and
   reacting a primary amine compound and the carbonyl halide.
[11] A method for producing a carbamoyl halide compound, the method comprising the steps of:
   producing a carbonyl halide by the method according to any one of the above [1] to [7], and
   reacting a secondary amine compound and the carbonyl halide.
[12] A method for producing an amino acid-N-carboxylic anhydride, the method comprising the steps of:
   producing a carbonyl halide by the method according to any one of the above [1] to [7], and
   reacting an amino acid compound represented by the following formula (VII) and the carbonyl halide,
   wherein the amino acid-N-carboxylic anhydride is represented by the following formula (VIII): 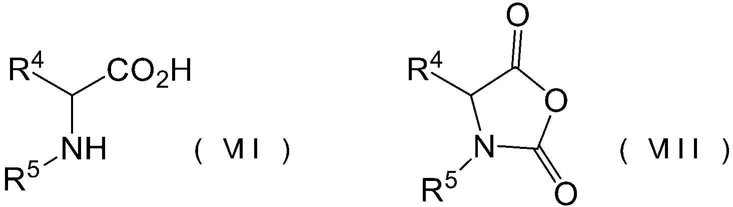 wherein
      R⁴ is an amino acid side chain group wherein a reactive group is protected,
      R⁵ is H or P¹-[-NH-CHR⁶-C(=O)-]ₗ- wherein R⁶ is an amino acid side chain group wherein a reactive group is protected, P¹ is a protective group of the amino group, 1 is an integer of 1 or more, and when 1 is an integer of 2 or more, a plurality of R⁶ may be the same as or different from each other.
[13] A method for producing a Vilsmeier reagent,
   wherein the Vilsmeier reagent is a salt represented by the following formula (X): 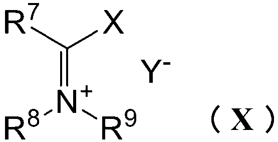 wherein
   R⁷ is a hydrogen atom, a C₁₋₆ alkyl group or an optionally substituted C₆₋₁₂ aromatic hydrocarbon group,
   R⁸ and R⁹ are independently a C₁₋₆ alkyl group or an optionally substituted C₆₋₁₂ aromatic hydrocarbon group, or R⁸ and R⁹ may form a 4 or more and 7 or less-membered ring structure together with each other,
   X is a halogeno group selected from the group consisting of chloro, bromo and iodo,
   Y⁻ is a counter anion,
   the method comprising the steps of:
      producing a carbonyl halide by the method according to any one of the above [1] to [7], and
      reacting the carbonyl halide and an amide compound represented by the following formula (IX): 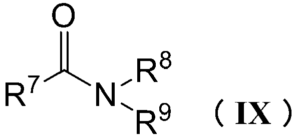
   wherein R⁷ to R⁹ have the same meanings as the above.
[14] A method for producing a urea compound, the method comprising the steps of:
   producing a carbonyl halide by the method according to any one of the above [1] to [7], and
   reacting a primary amine compound or a secondary amine compound and the carbonyl halide.

### EFFECT OF THE INVENTION

Methane, which is inexpensive and of which global warming potential is higher than that of carbo dioxide, can be used as a raw material, and thus a carbonyl halide can be produced at a lower cost and a lower environmental load by the present invention method. In addition, though the reaction of methane in the presence of oxygen may possibly cause explosion, the reaction may rapidly proceed and a carbonyl halide can be safely produced by irradiating light to a mixed gas containing methane, a halogen molecule gas and oxygen. Further, a carbonyl halide cannot be leaked out or can be hardly leaked out from the system by supplying the produced carbonyl halide to be reacted into a solution containing a reactive substrate compound in the same system and the supplying the gas after the reaction into a trap in the present invention. Thus, the present invention is industrially useful, since a carbonyl halide such as carbonyl chloride can be safely produced at a low cost and a low environmental load, and furthermore the compound produced from a carbonyl halide, such as a carbonate derivative, can be produced safely and efficiently at a low cost and a low environmental load.

### BRIEF DESCRIPTION OF THE DRAWINGS

[Fig. 1] Fig. 1 is a schematic diagram to demonstrate one example of the constitution of a reaction apparatus usable in the present invention.
[Fig. 2] Fig. 2 is the result of the analysis of the reaction gas according to the present invention by ¹³C NMR.

### MODE FOR CARRYING OUT THE INVENTION

The present invention method is hereinafter described step by step, and the present invention is not restricted to the following specific examples.

### · Step to irradiate light

Light is irradiated to a mixed gas comprising methane, a halogen molecule gas and oxygen to produce a carbonyl halide in this step. A carbonyl halide is also referred to as carbonyl dihalide. For example, carbonyl chloride is referred to as carbonyl dichloride.

The boiling point of methane is -161.6°C, and methane is gas under atmospheric temperature and atmospheric pressure. Thus, for example, the methane derived from a methane gas cylinder may be directly used. Alternatively, an organic substance such as garbage, paper waste and manure of livestock is subjected to methane fermentation using methane bacteria to produce methane gas with low environmental load and the methane gas may be used, and the methane derived from methane hydrate may be used. Further, since the most part of city gas is methane gas, city gas can be directly used or the methane gas purified from city gas can be used. A method for producing methane from carbon dioxide and hydrogen has been developed, and the methane produced by the method can be also used.

Biogas may be used as the raw material gas containing methane. Biogas means the gas generated by methane fermentation of excretion of an organism such as livestock, food waste, sludge derived from sewage, paper waste, plant waste or an energy crop. The main components of biogas are methane and carbon dioxide. For example, biogas contains 50 to 75 vol% of methane and 25 to 50 vol% of carbon dioxide and additionally nitrogen, hydrogen, hydrogen sulfide, oxygen and siloxane in some cases. Since biogas can be produced by methane fermentation of biological resources and can fix carbon by the present invention, biogas can be considered as carbon-negative. The biogas generated by anaerobic fermentation of sewage sludge is called as digester gas, and such digester gas can be also used in the present invention.

City gas can be used as the raw material gas containing methane. City gas contains methane gas as the main component, is more inexpensive than purified methane gas, and does not contain or hardly contains the component that inhibits the reaction of the present invention method. For example, the component composition of a certain city gas product is about 88.9 vol% of methane, about 6.8 vol% of ethane, about 3.1 vol% of propane and about 1.2 vol% of butane.

The usage amount of methane may be appropriately adjusted in the range that a sufficient amount of carbonyl halide can be produced. For example, 0.1 times or more by mole of methane to the reactant compound to be reacted with the produced carbonyl halide may be used. The upper limit of the usage amount of methane is not particularly restricted. For example, the usage amount may be adjusted to 200 times or less by mole to the reactant compound. The usage amount is preferably 1 time or more by mole, 5 times or more by mole or 10 times or more by mole, more preferably 20 times or more by mole, even more preferably 25 times or more by mole, and preferably 150 times or less by mole, more preferably 100 times or less by mole, even more preferably 50 times or less by mole.

Gaseous halogen molecule is used in the present invention. An example of halogen molecule includes chlorine molecule, bromine molecule and iodine molecule. Since the boiling point of bromine molecule is 58.8°C and iodine molecule has to be sublimed, chlorine molecule and bromine molecule are preferred and chlorine molecule is more preferred.

A commercially available gas cylinder of a halogen molecule gas may be used as the source to supply a halogen molecule gas, and a halogen molecule gas may be prepared at the time of use. For example, chlorine molecule can be produced by adding concentrated hydrochloric acid to manganese oxide (IV) and heating the mixture, or adding concentrated hydrochloric acid to calcium hypochlorite, and is industrially produced by the electrolysis of sodium chloride aqueous solution.

MnO₂ + 4HCl → MnCl₂ + 2H₂O + Cl₂

Ca(ClO)₂·3H₂O + 4HCl → CaCl₂ + 5H₂O + 2Cl₂

2NaCl + 2H₂O → 2NaOH + H₂ + Cl₂

Bromine molecule can be produced by adding KClO₃ and H₂SO₄ to a Group 2 metal salt of bromide ion and heating the mixture, or flowing a Group 2 metal salt of bromide ion downward and countercurrently introducing a chlorine molecule gas from below.

3MgBr₂ + 3H₂SO₄ + KClO₃ → 3MgSO₄ + KCl + 3Br₂ + 3H₂O

MgBr₂ + Cl₂ → MgCl₂ + Br₂

Iodine molecule can be produced by blowout method or ion-exchange method. Easily vaporized iodine molecule is extruded from brine containing iodide ion using air in blowout method. Iodide ion is selectively adsorbed on an ion-exchange resin to be concentrated in ion-exchange method.

The usage amount of the halogen molecule gas may be appropriately adjusted as long as a sufficient amount of carbonyl halide can be produced. For example, 0.5 times or more by mole and 5 times or less by mole of the halogen molecule may be used to 1 mole of methane. The ratio is preferably 0.8 times or more by mole, more preferably 1 time or more by mole, and preferably 3 times or less by mole, preferably 2 times or less by mole.

The mechanism to accelerate the reaction by the halogen molecule gas is not necessarily clear, and a halogen oxide may be produced by the photoreaction of the halogen molecule and oxygen, and the halogen oxide may be reacted with methane. An example of a chlorine oxide includes chlorine monoxide, chlorine dioxide and dichlorine heptoxide.

The source of oxygen may be a gas containing oxygen. For example, air and purified oxygen may be used. Purified oxygen may be mixed with an inactive gas such as nitrogen and argon to be used. Air may be used in terms of a cost and easiness. The risk of explosion can be further reduced by using air. For example, the oxygen content percentage in an oxygen-containing gas may be adjusted to 15 vol% or more and 100 vol% or less. Since an excessive oxidation reaction of methane may be suppressed by reducing the oxygen content percentage, the oxygen content percentage is preferably 75 vol% or less or 50 vol% or less and more preferably 30 vol% or less. In addition, air is preferably used. For example, the oxygen content percentage in the oxygen-containing gas can be adjusted by an inert gas such as nitrogen, carbon dioxide and argon. Substantially only oxygen is also preferably used except for an unavoidable impurity. A method for supplying the oxygen-containing gas is not particularly restricted, and the oxygen-containing gas may be supplied into the reaction system from an oxygen cylinder equipped with a mass flow controller or supplied into the reaction system from an oxygen generator.

The usage amount of oxygen may be appropriately adjusted as long as a sufficient amount of carbonyl halide can be produced. For example, the amount thereof to 1 mole of methane may be adjusted to 0.1 times or more by mole and 5 times or less by mole. The ratio is preferably 0.2 times or more by mole, more preferably 0.5 times or more by mole, and preferably 4 times or less by mole or 2 times or less by mole, preferably 1.5 times or less by mole or 1 time or less by mole.

Ozone may be produced by irradiating light on oxygen, and the ozone may be involved in the reaction. Ozone may be used in addition to oxygen.

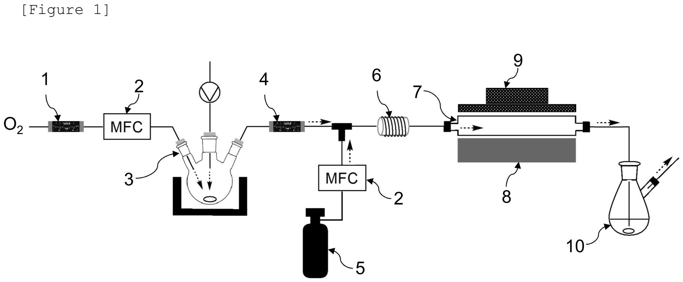

The mixed gas comprising methane, a halogen molecule gas and oxygen may be prepared in a photoreaction vessel to which light is applied by supplying each raw material gas into the photoreaction vessel in a batch manner or a continuous manner. Two or more raw material gasses selected from methane, a halogen molecule gas and oxygen may be preliminarily mixed to be supplied into a photoreaction vessel. For example, a mixed gas is prepared by preliminarily mixing an oxygen-containing gas and chlorine gas, and further mixing methane gas therein in the exemplary embodiment demonstrated in Figure 1.

The concentration of methane in the mixed gas may be appropriately adjusted. For example, the concentration may be adjusted to 5 vol% or more and 30 vol% or less. When the concentration is 5 vol% or more, a carbonyl halide can be produced more efficiently. When the concentration is 30 vol% or less, the excessive decomposition of methane can be suppressed more surely, and a carbonyl halide can be produced with higher yield. The concentration is preferably 10 vol% or more and 25 vol% or less.

Since a carbonyl halide may be reacted with water to be decomposed into carbon dioxide and a hydrogen halide, the raw material gas is preferably preliminarily dried in the case where the raw material gas may contain water. For example, the halogen molecule gas prepared at time of use and air is preferably dried using a desiccant such as silica gel, calcium chloride, magnesium chloride and calcium oxide.

When each raw material gas is continuously supplied, a ratio of the usage amount of each raw material gas can be adjusted by the flow rate of each raw material gas to be supplied into a photoreaction vessel. The flow rate of the mixed gas in a photoreaction vessel that is used for irradiating light to the mixed gas is preferably determined in consideration of the internal volume of the photoreaction vessel. For example, when the internal volume of a photoreaction vessel is large, the flow rate is preferably adjusted to be faster, since the residence time of the mixed gas tends to be long. On the one hand, when the internal volume of a photoreaction vessel is small, the flow rate of the mixed gas is preferably adjusted to be slower. Specifically, since internal volume of photoreaction vessel (L)/flow rate of mixed gas (L/s) corresponds to a residence time (s) of the mixed gas in a photoreaction vessel, the flow rate of the mixed gas may be determined on the basis of a desired residence time and the internal volume of a photoreaction vessel. The linear speed of the mixed gas in a photoreaction vessel may be adjusted to about 0.001 m/min or more and 100 m/min or less. When the linear speed is 0.001 m/min or more, the photodecomposition of the produced carbonyl halide can be suppressed more surely. When the linear speed is 100 m/min or less, sufficient time for the conversion of methane, a halogen molecule and oxygen to a carbonyl halide can be ensured more reliably. The linear speed can be calculated by dividing the flow rate of the mixed gas passing through a photoreaction vessel by the cross-sectional area of the inside of the photoreaction vessel. When the cross-sectional area of the inside of a photoreaction vessel is not uniform, the cross-sectional area may be regarded as the average value of the cross-sectional areas of the photoreaction vessel in the moving direction of the mixed gas. The average value can be calculated by dividing the volume of the inside of a photoreaction vessel by the length of the photoreaction vessel in the moving direction of the mixed gas. The linear speed is preferably 0.01 m/min or more, and preferably 50 m/min or less or 20 m/min or less, more preferably 10 m/min or less or 5 m/min or less, even more preferably 1 m/min or less or 0.5 m/min or less.

The light comprising a high energy light comprising a short wavelength can be used as the light to be irradiated to the mixed gas. For example, the light comprising ultraviolet light can be used. A carbonyl halide may be efficiently produced by the high energy light comprising a short wavelength. Specifically, the light comprising the light comprising the wavelength of 180 nm or more and 500 nm or less and the light having the peak wavelength of 180 nm or more and 500 nm or less can be used. The peak wavelength means the wavelength having the highest intensity. The wavelength of the light to be irradiated may be appropriately determined. The light having the wavelength of 400 nm or less can be used, the light having the wavelength of 300 nm or less can be used, and the light of which peak wavelength included in the ranges can be also used. For example, the light comprising UV-B having the wavelength of 280 nm or more and 315 nm or less and/or UV-C having the wavelength of 180 nm or more and 280 nm or less can be used, the light comprising UV-C having the wavelength of 180 nm or more and 280 nm or less can be used, and the light of which peak wavelength is included in the ranges can be also used.

The safe light having relatively low energy may be also used in the present invention in order to suppress the decomposition of the produced carbonyl halide. An example of the light to be irradiated having relatively low energy includes the light of which peak wavelength is included in a visible light wavelength range.

Visible light specifically means the light having the peak wavelength of 360 nm or more and 830 nm or less. High energy light such as ultraviolet light may be contained in the spectral distribution of the light to be irradiated in the present invention method, and ultraviolet light has less impact as long as the peak wavelength of a spectral distribution includes in the above range. The peak wavelength means the wavelength of which intensity is the strongest in the spectral distribution of the light. The peak wavelength is preferably 400 nm or more, and preferably 800 nm or less or 700 nm or less, more preferably 600 nm or less. High energy light such as ultraviolet light can be blocked by using a reaction vessel composed of borosilicate glass such as Pyrex (registered trademark) glass.

A chlorine gas among the halogen molecule gas used in the present invention is highly convenient, since a chlorine gas exists as a gas under atmospheric temperature and atmospheric pressure. A chlorine gas particularly absorbs the light of 280 nm or more and less than 360 nm. Thus, the light having the wavelength of 280 nm or more and less than 360 nm is also preferred as the light to be irradiated. When a peak wavelength is included in the range of 360 nm or more and 830 nm or less, the light may be safe even in the case where the light comprises the light having the wavelength of 280 nm or more and less than 360 nm.

A means for the light irradiation is not particularly restricted as long as the light having the above-described wavelength can be irradiated by the means. An example of a light source of the light having such a wavelength range includes sunlight, low pressure mercury lamp, medium pressure mercury lamp, high pressure mercury lamp, ultrahigh pressure mercury lamp, chemical lamp, black light lamp, metal halide lamp and LED lamp. A low pressure mercury lamp and an LED lamp are preferred and an LED lamp is more preferred in terms of reaction efficiency and a cost.

The conditions such as a strength of the irradiation light and an irradiation time may be appropriately determined depending on the kind and the usage amount of the starting raw material. For example, the light strength at the shortest distance position of the mixed gas from the light source is preferably 1 mW/cm² or more and 100 mW/cm² or less. The light irradiation time is preferably 1 minute or more and 10 hours or less, more preferably 5 minutes or more and 5 hours or less, and even more preferably 10 minutes or more and 1 hour or less. The embodiment to irradiate light is not particularly restricted, and any embodiments can be adopted. For example, light may be continuously irradiated from the start to the end of the reaction, the light irradiation and the light non-irradiation may be alternately repeated, or light may be irradiated during a predetermined time only from the start of the reaction. Light is preferably continuously irradiated from the supply of the reaction gas into a photoreaction vessel to the discharge of the reaction gas. The shortest distance between the light source and the mixed gas is preferably 1 m or less, more preferably 50 cm or less, and even more preferably 10 cm or less or 5 cm or less. The lower limit of the shortest distance is not particularly restricted and may be 0 cm. In other words, the light source is placed in a photoreaction vessel or the mixed gas. When light is irradiated from the side face of a reaction vessel, the shortest distance may be 1 cm or more or 2 cm or more.

The temperature for the photoreaction is not particularly restricted and may be appropriately adjusted. For example, the temperature may be adjusted to -20°C or higher and 200°C or lower. The temperature is more preferably -10°C or higher, even more preferably 0°C or higher or 10°C or higher, and more preferably 150°C or lower or 100°C or lower, even more preferably 60°C or lower or 35°C or lower. In addition, the reaction may be conducted under atmospheric temperature without adjusting temperature.

A carbonyl halide [X-C(=O)-X wherein X is one or more halogeno groups selected from the group consisting of chloro, bromo and iodo.] may be produced by irradiating light to the mixed gas. When there is a reactive substrate compound in the reaction system, a carbonyl halide-like compound which plays a similar role to a carbonyl halide may be also reacted with the reactive substrate compound in addition to the produced carbonyl halide. The carbonyl halide-like compound is included in the carbonyl halide of the present invention. The representative reactions in which the carbonyl halide is used are hereinafter described.

An example of a reaction device usable for the present invention includes a reaction vessel equipped with a means for irradiating light. Figure 1 is an embodiment of a reaction apparatus usable for the production method of the present invention. A mixed gas of a halogen molecule gas and an oxygen-containing gas is produced in the reaction apparatus demonstrated as Figure 1 by producing the halogen molecule gas using the halogen molecule gas generator 3, drying an oxygen-containing gas using the silica gel drying tube 1, and supplying the dried oxygen-containing gas to the halogen molecule gas generator 3. The produced mixed gas is dried using the calcium chloride drying tube 4 and then mixed with a methane gas to produce a mixed gas. The mixed gas may be heated using the coil heater 6. The flow rate of the mixed gas can be adjusted by the mass flow controller 2. The mixed gas is supplied into the photoreaction vessel 7, and light is irradiated thereto from the light source 9. The temperature for the photoreaction in the photoreaction vessel 7 can be adjusted by the heater 8. The reaction gas discharged from the photoreaction vessel 7 contains a carbonyl halide. The carbonyl halide can be reacted with a reactive substrate compound by blowing the reaction gas into a solution containing the reactive substrate compound in the reaction vessel 10. The gas discharged from the reaction vessel 10 may be supplied into a trap to capture and/or decompose the excessive carbonyl halide. The production and the consumption of a carbonyl halide and the production of a useful compound can be conducted in one system by the reaction apparatus, and the leakage of a carbonyl halide out of the system can be suppressed.

### · Neutralization step

A sodium halide aqueous solution is obtained in this step by neutralizing a hydrogen halide with a basic sodium salt. The hydrogen halide is produced as a side product by irradiating light to the mixed gas.

A hydrogen halide may be produced from methane in accordance with the following reaction formula in the present invention method.

CH₄ + 3X₂ + 1/2O₂ → X-C(=O)-X + 4HX

In addition, when the produced carbonyl halide is used for a reaction, a hydrogen halide may be produced.

The hydrogen halide produced as a side product may be purified or roughly purified to be used as a product of a hydrogen halide or an aqueous solution thereof. On the one hand, a sodium halide aqueous solution is prepared in this step by neutralizing the hydrogen halide produced as a side product in the present invention method or in the reaction of the carbonyl halide produced by the present invention method. For example, the efficiency of the present invention method can be further improved by using the sodium halide aqueous solution produced in this step as all or a part of the sodium halide aqueous solution used in the step to produce a halogen molecule gas described later.

A basic sodium salt means a sodium ion salt of which aqueous solution shows basicity. An example of the basic sodium salt includes sodium hydroxide, sodium hydrogencarbonate, sodium carbonate, sodium acetate, disodium phosphate and sodium citrate. The basic sodium salt is preferably selected from sodium hydroxide, sodium hydrogencarbonate and sodium carbonate.

The condition for performing this step may be suitably determined. For example, the hydrogen halide produced as a side product in the present invention method or the hydrogen halide produced as a side produce by the reaction of the carbonyl halide produced by the present invention method may be neutralized by adding the hydrogen halide to an aqueous solution of the basic sodium salt. A sodium halide and water are produced by the reaction of the basic sodium salt and a hydrogen halide, and the produced sodium halide aqueous solution can be utilized in the step described later to produce a halogen molecule gas.

### · Step to produce halogen molecule gas

A halogen molecule gas, which is one of raw material gases of the above-described light irradiation step, is produced in this step by the electrolysis of a sodium halide aqueous solution. The execution of this step is optional. For example, a halogen molecule gas product may be purchased to be used, but relatively dangerous halogen molecule gas, which is toxic and caustic, can be produced in situ by this step and thus the risk of accidents caused by transporting a halogen molecule gas can be reduced. When a chlorine gas is used as a halogen molecule gas, the execution cost of this step can be significantly reduced by using seawater as a sodium chloride aqueous solution.

The electrolysis of a sodium halide aqueous solution may be conventionally executed. For example, titanium, platinum or the like can be used as an electrode metal. Direct current electricity can be used as current of electricity. A halogen molecule is produced at an anode, and sodium hydroxide is produced at a cathode, in the electrolysis of a sodium halide aqueous solution. Since sodium hypohalite (NaOX) may be produced in some cases by reacting a halogen molecule with sodium hydroxide, ultrasonic may be irradiated to a sodium halide aqueous solution or both electrodes may be separated by a separator during the electrolysis.

A commercially available sodium halide aqueous solution may be used, or a sodium halide aqueous solution may be prepared by dissolving a commercially available sodium halide in water. On the one hand, the efficiency of the present invention method can be further improved by using the basic sodium salt produced by the above-described neutralization step.

Reaction examples of a carbonyl halide produced by the present invention method with a reactive substrate compound are hereinafter described.

### · Post-reaction step - production of carbonate compound

A carbonate compound can be produced by reacting the carbonyl halide and an alcohol compound. The embodiment of the reaction is not particularly restricted. For example, the gas containing the produced carbonyl halide may be blown into a composition containing an alcohol compound in the reaction vessel 10 as demonstrated in Figure 1.

An alcohol compound means an organic compound having a hydroxy group. An example thereof includes a monovalent alcohol compound represented by the following formula (I) and a divalent alcohol compound represented by the following formula (II). Hereinafter, the compound represented by formula x is abbreviated as "compound x" in some cases. For example, a "monovalent alcohol compound represented by the formula (I)" is abbreviated as the "monovalent alcohol compound (I)" in some cases.

R¹-OH ··· (I)

HO-R²-OH ··· (II)

wherein R¹ is a monovalent organic group, and R² is a divalent organic group.

An organic group is not particularly restricted as long as the organic group is inactive in the reaction of this step. An example thereof includes an optionally substituted C₁₋₁₀ aliphatic hydrocarbon group, an optionally substituted C₆₋₃₀ aromatic hydrocarbon group, an optionally substituted heteroaryl group, an organic group formed by binding 2 or more and 5 or less of an optionally substituted C₁₋₁₀ aliphatic hydrocarbon group and an optionally substituted C₆₋₁₂ aromatic hydrocarbon group, and an organic group formed by binding 2 or more and 5 or less of an optionally substituted C₁₋₁₀ aliphatic hydrocarbon group and an optionally substituted heteroaryl group.

An example of the C₁₋₁₀ aliphatic hydrocarbon group includes a C₁₋₁₀ chain aliphatic hydrocarbon group, a C₃₋₁₀ cyclic aliphatic hydrocarbon group, and an organic group formed by binding 2 or more and 5 or less of a C₁₋₁₀ chain aliphatic hydrocarbon group and a C₃₋₁₀ cyclic aliphatic hydrocarbon group.

The "C₁₋₁₀ chain aliphatic hydrocarbon group" means a linear or branched saturated or unsaturated aliphatic hydrocarbon group having the carbon number of 1 or more and 10 or less. An example of the monovalent C₁₋₁₀ chain aliphatic hydrocarbon group includes a C₁₋₁₀ alkyl group, a C₂₋₁₀ alkenyl group and a C₂₋₁₀ alkynyl group.

An example of the C₁₋₁₀ alkyl group includes methyl, ethyl, n-propyl, isopropyl, n-butyl, 1-methylpropyl, 2-methylpropyl, 1,1-dimethylethyl, 2,2-dimethylethyl, n-pentyl, n-hexyl, 2-hexyl, 3-hexyl, 4-methyl-2-pentyl, n-heptyl, n-octyl and n-decyl. The C₁₋₁₀ alkyl group is preferably a C₁₋₆ alkyl group, more preferably a C₁₋₄ alkyl group and even more preferably methyl.

An example of the C₂₋₁₀ alkenyl group includes ethenyl (vinyl), 1-propenyl, 2-propenyl (allyl), butenyl, hexenyl, octenyl and decenyl. The C₂₋₁₀ alkenyl group is preferably a C₂₋₈ alkenyl group and more preferably a C₄₋₆ alkenyl group.

An example of the C₂₋₁₀ alkynyl group includes ethinyl, propynyl, butynyl, hexynyl, octynyl and pentadecynyl. The C₂₋₁₀ alkynyl group is preferably a C₂₋₈ alkynyl group and more preferably a C₂₋₆ alkynyl group.

The "C₃₋₁₀ cyclic aliphatic hydrocarbon group" means a cyclic saturated or unsaturated aliphatic hydrocarbon group having the carbon number of 3 or more and 10 or less. An example of the monovalent C₃₋₁₀ cyclic aliphatic hydrocarbon group includes a C₃₋₁₀ cycloalkyl group, a C₄₋₁₀ cycloalkenyl group and a C₄₋₁₀ cycloalkynyl group.

An example of the organic group formed by binding 2 or more and 5 or less of the C₁₋₁₀ chain aliphatic hydrocarbon group and the C₃₋₁₀ cyclic aliphatic hydrocarbon group includes a monovalent C₃₋₁₀ cyclic aliphatic hydrocarbon group-divalent C₁₋₁₀ chain aliphatic hydrocarbon group, and a monovalent C₁₋₁₀ chain aliphatic hydrocarbon group-divalent C₃₋₁₀ cyclic aliphatic hydrocarbon group-divalent C₁₋₁₀ chain aliphatic hydrocarbon group.

The "C₆₋₁₂ aromatic hydrocarbon group" means an aromatic hydrocarbon group having the carbon number of 6 or more and 12 or less. An example of the monovalent C₆₋₁₂ aromatic hydrocarbon group includes phenyl, indenyl, naphthyl and biphenyl, and phenyl is preferred.

The "C₆₋₃₀ aromatic hydrocarbon group" means an aromatic hydrocarbon group having the carbon number of 6 or more and 30 or less. An example of the divalent C₆₋₃₀ aromatic hydrocarbon group includes an aromatic hydrocarbon group in the alcohol compound (II-1) described later in addition to a divalent C₆₋₁₂ aromatic hydrocarbon group such as phenylene, indenylene, naphthylene and biphenylene.

The "heteroaryl group" means a 5-membered aromatic heterocyclic group, a 6-membered aromatic heterocyclic group and a condensed ring aromatic heterocyclic group having at least one hetero atom such as a nitrogen atom, an oxygen atom and a sulfur atom. An example thereof includes a monovalent 5-membered aromatic heterocyclic group such as pyrrolyl, imidazolyl, pyrazolyl, thienyl, furyl, oxazolyl, isoxazolyl, thiazolyl, isothiazolyl and thiadiazolyl; a monovalent 6-membered aromatic heterocyclic group such as pyridinyl, pyrazinyl, pyrimidinyl and pyridazinyl; and a monovalent condensed ring aromatic heterocyclic group such as indolyl, isoindolyl, quinolinyl, isoquinolinyl, benzofuranyl, isobenzofuranyl and chromenyl.

An example of the "organic group formed by binding 2 or more and 5 or less of the C₁₋₁₀ aliphatic hydrocarbon group and the C₆₋₁₂ aromatic hydrocarbon group" includes a C₆₋₁₂ aromatic hydrocarbon group-C₁₋₁₀ chain aliphatic hydrocarbon group, a C₁₋₁₀ chain aliphatic hydrocarbon group-C₆₋₁₂ aromatic hydrocarbon group, a C₁₋₁₀ chain aliphatic hydrocarbon group-C₆₋₁₂ aromatic hydrocarbon group-C₁₋₁₀ chain aliphatic hydrocarbon group, and a C₆₋₁₂ aromatic hydrocarbon group-C₁₋₁₀ chain aliphatic hydrocarbon group-C₆₋₁₂ aromatic hydrocarbon group. An example of the "organic group formed by binding 2 or more and 5 or less of the C₁₋₁₀ aliphatic hydrocarbon group and the heteroaryl group" includes a heteroaryl group-C₁₋₁₀ chain aliphatic hydrocarbon group, a C₁₋₁₀ chain aliphatic hydrocarbon group-heteroaryl group, a C₁₋₁₀ chain aliphatic hydrocarbon group-heteroaryl group-C₁₋₁₀ chain aliphatic hydrocarbon group, and a heteroaryl group-C₁₋₁₀ chain aliphatic hydrocarbon group-heteroaryl group.

An example of the substituent group that the C₁₋₁₀ aliphatic hydrocarbon group may optionally have includes one or more substituent groups selected from the group consisting of a halogeno group, a nitro group and a cyano group, and a halogeno group is preferred. An example of the substituent group that the C₆₋₁₂ aromatic hydrocarbon group and the heteroaryl group may optionally have includes one or more substituent groups selected from the group consisting of a C₁₋₆ alkyl group, a C₁₋₆ alkoxy group, a halogeno group, a nitro group and a cyano group, and a halogeno group is preferred. An example of the halogeno group includes fluoro, chloro, bromo and iodo, and fluoro is preferred.

An alcohol compound may be classified into a fluorinated alcohol compound and a non-fluorinated alcohol compound. The fluorinated alcohol compound necessarily has a fluoro group as a substituent group, and the non-fluorinated alcohol compound is not substituted with a fluoro group. The halogeno group that a non-fluorinated alcohol compound may optionally have is one or more halogeno groups selected from chloro, bromo and iodo. The group "R^{x}" having a fluoro as a substituent group may be described as "R_{F}^{x}".

The "C₁₋₆ alkyl group" means a monovalent linear or branched saturated aliphatic hydrocarbon group having the carbon number of 1 or more and 6 or less. An example thereof includes methyl, ethyl, n-propyl, isopropyl, n-butyl, isobutyl, s-butyl, t-butyl, n-pentyl and n-hexyl. The C₁₋₆ alkyl group is preferably a C₁₋₄ alkyl group, more preferably a C₁₋₂ alkyl group and even more preferably methyl.

The "C₁₋₆ alkoxy group" means a linear or branched saturated aliphatic hydrocarbon oxy group having the carbon number of 1 or more and 6 or less. An example thereof includes methoxy, ethoxy, n-propoxy, isopropoxy, n-butoxy, isobutoxy, t-butoxy, n-pentoxy and n-hexoxy. The C₁₋₆ alkoxy group is preferably a C₁₋₄ alkoxy group, more preferably a C₁₋₂ alkoxy group and even more preferably methoxy.

The monovalent alcohol compound (I) may be a monovalent fluorinated alcohol compound. An example of the monovalent fluorinated alcohol compound (I) includes a fluorinated ethanol such as difluoroethanol and trifluoroethanol; and a fluorinated propanol such as monofluoropropanol, difluoropropanol, trifluoropropanol, tetrafluoropropanol, pentafluoropropanol and hexafluoropropanol.

An example of the divalent organic group includes divalent organic groups derived from the examples of the monovalent organic group. For example, the divalent organic group derived from the C₁₋₁₀ alkyl group, the C₂₋₁₀ alkenyl group and the C₂₋₁₀ alkynyl group as the monovalent organic group is a C₁₋₁₀ alkane-diyl group, a C₂₋₁₀ alkene-diyl group and a C₂₋₁₀ alkyne-diyl group.

The divalent organic group may be a divalent (poly)alkylene glycol group: -[-O-R²-]ₙ- wherein R² is a C₁₋₈ alkane-diyl group, and n is an integer of 1 or more and 50 or less.

In addition, an example of the divalent alcohol compound (II) includes the following divalent alcohol compound (II-1): 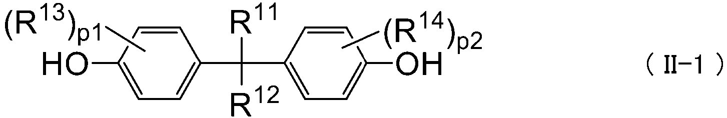 wherein
R¹¹ and R¹² are independently H, a C₁₋₆ alkyl group, a C₁₋₆ fluoroalkyl group or a C₆₋₁₂ aromatic hydrocarbon group, or form a C₃₋₆ cycloalkyl group optionally substituted with a C₁₋₆ alkyl together with each other,
R¹³ and R¹⁴ are independently H, a C₁₋₆ alkyl group or a C₆₋₁₂ aromatic hydrocarbon group, and when p1 or p2 is an integer of 2 or more, a plurality of R¹³ or R¹⁴ are the same as or different from each other,
p1 and p2 are independently integers of 0 or more and 4 or less.

An example of the specific divalent non-fluorinated alcohol compound (II-1) includes 2,2-bis(4-hydroxyphenyl)propane, 1,1-bis(4-hydroxyphenyl)-1-phenylethane, 2,2-bis(4-hydroxyphenyl)hexafluoropropane, 2,2-bis(4-hydroxyphenyl) butane, bis(4-hydroxyphenyl)diphenylmethane, 2,2-bis(3-methyl-4-hydroxyphenyl)propane, 1,1-bis(4-hydroxyphenyl) ethane, bis(4-hydroxyphenyl)methane and 2,2-bis(4-hydroxy-3-isopropylphenyl)propane, and 2,2-bis(4-hydroxyphenyl) propane, i.e. Bisphenol A, is preferred.

The divalent alcohol compound (II) may be a divalent fluorinated alcohol compound. An example of such a divalent fluorinated alcohol compound (II) includes fluorinated ethylene glycol; a fluorinated propylene glycol such as monofluoropropylene glycol and difluoropropylene glycol; a fluorinated butanediol such as monofluorobutanediol, difluorobutanediol, trifluorobutanediol and tetrafluorobutanediol; a fluorinated pentanediol such as monofluoropentanediol, difluoropentanediol, trifluoropentanediol, tetrafluoropentanediol, pentafluoropentanediol and hexafluoropentanediol; a fluorinated hexanediol such as monofluorohexanediol, difluorohexanediol, trifluorohexanediol, tetrafluorohexanediol, pentafluorohexanediol, hexafluorohexanediol, heptafluorohexanediol and octafluorohexanediol; a fluorinated heptanediol such as monofluoroheptanediol, difluoroheptanediol, trifluoroheptanediol, tetrafluoroheptanediol, pentafluoroheptanediol, hexafluoroheptanediol, heptafluoroheptanediol, octafluoroheptanediol, nonafluoroheptanediol and decafluoroheptanediol; a fluorinated octanediol such as monofluorooctanediol, difluorooctanediol, trifluorooctanediol, tetrafluorooctanediol, pentafluorooctanediol, hexafluorooctanediol, heptafluorooctanediol, octafluorooctanediol, nonafluorooctanediol, decafluorooctanediol, undecafluorooctanediol and dodecafluorooctanediol; a fluorinated nonanediol such as monofluorononanediol, difluorononanediol, trifluorononanediol, tetrafluorononanediol, pentafluorononanediol, hexafluorononanediol, heptafluorononanediol, octafluorononanediol, nonafluorononanediol, decafluorononanediol, undecafluorononanediol, dodecafluorononanediol, tridecafluorononanediol and tetradecafluorononanediol; a fluorinated decanediol such as monofluorodecanediol, difluorodecanediol, trifluorodecanediol, tetrafluorodecanediol, pentafluorodecanediol, hexafluorodecanediol, heptafluorodecanediol, octafluorodecanediol, nonafluorodecanediol, decafluorodecanediol, undecafluorodecanediol, dodecafluorodecanediol, tridecafluorodecanediol, tetradecafluorodecanediol, pentadecafluorodecanediol and hexadecafluorodecanediol; and a fluorinated polyethylene glycol such as fluorinated diethylene glycol, fluorinated triethylene glycol, fluorinated tetraethylene glycol, fluorinated pentaethylene glycol and fluorinated hexaethylene glycol.

The usage amount of the alcohol compound may be appropriately adjusted as long as the reaction proceeds successfully. For example, 1 or more molar ratio of the divalent alcohol compound to the produced carbonyl halide may be used, and 2 or more molar ratio of the monovalent alcohol compound to the produced carbonyl halide may be used. A carbonate compound can be produced more efficiently by using excessive amount of the alcohol compound. Since the yield of the carbonyl halide to the used methane is not constant, the molar ratio of the divalent alcohol compound to the methane is preferably adjusted to 1 or more and the molar ratio of the monovalent alcohol compound to the methane is preferably adjusted to 2 or more. The molar ratio of the divalent alcohol is preferably 1.5 or more, more preferably 2 or more, and preferably 10 or less, more preferably 5 or less. The molar ratio of the monovalent alcohol is preferably 2.5 or more, more preferably 4 or more, and preferably 20 or less, more preferably 10 or less.

A base may be used for accelerating the reaction of the carbonyl halide with the alcohol compound. A base is classified into an inorganic base and an organic base. An example of the inorganic base includes a carbonate salt of an alkali metal, such as lithium carbonate, sodium carbonate, potassium carbonate and cesium carbonate; a carbonate salt of a Group 2 metal, such as magnesium carbonate, calcium carbonate and barium carbonate; a hydrogencarbonate salt of an alkali metal, such as lithium hydrogencarbonate, sodium hydrogencarbonate, potassium hydrogencarbonate and cesium hydrogencarbonate; a hydroxide of an alkali metal, such as lithium hydroxide, sodium hydroxide and potassium hydroxide; a hydroxide of a Group 2 metal, such as magnesium hydroxide and calcium hydroxide; a fluoride salt of an alkali metal, such as lithium fluoride, sodium fluoride, potassium fluoride and cesium fluoride. A carbonate salt or a hydrogencarbonate salt of an alkali metal or a Group 2 metal is preferred due to relatively low moisture absorbency and relatively low deliquescency, and a carbonate salt of an alkali metal is more preferred. For example, a tri(C₁₋₄ alkyl)amine such as trimethylamine, triethylamine and diisopropylethylamine; a tert-butoxide of an alkali metal, such as sodium tert-butoxide and potassium tert-butoxide; and a non-nucleophilic organic base such as diazabicycloundecene, lithium diisopropylamide, lithium tetramethylpiperidine, 1,4-diazabicyclo[2.2.2]octane (DABCO), 1,5,7-triazabicyclo[4.4.0]dec-5-ene (TBD), 7-methyl-1,5,7-triazabicyclo[4.4.0]dec-5-ene (MTBD), 1,8-diazabicyclo[5.4.0]undec-7-ene (DBU), 1,5-diazabicyclo[4.3.0]non-5-ene (DBN), 1,1,3,3-tetramethylguanidine (TMG) and N-methylmorpholine can be used as an organic base in terms of low reactivity with the product by the photoreaction of tetrahaloethylene. Also, low-nucleophilic organic base such as pyridine and lutidine may be used.

A hydrogen halide such as hydrogen chloride is produced as a side product during the photoreaction and the reaction of a carbonyl halide with an alcohol compound. A base is useful for capturing such a hydrogen halide. But when a reaction tube having a small diameter is used, a salt of a hydrogen halide and a base may precipitate and thus the reaction tube may become clogged in some cases. The base of which salt with a hydrogen halide is an ionic liquid is preferably used in such a case. An example of such a base includes an organic base such as an imidazole derivative, for example, 1-methylimidazole. In addition, the base of which hydrochloride salt has a relatively low melting point, such as pyridine, may be used.

The usage amount of the base may be appropriately adjusted as long as the reaction proceeds successfully. For example, the usage amount to 1 mole of methane may be adjusted to 1 mole or more and 10 mole or less.

For example, the base may be added to the alcohol compound, or the base may be continuously supplied with the alcohol compound.

When the carbonyl halide and the alcohol compound are reacted, a solvent may be used. For example, a solvent may be added to the composition containing the alcohol compound. An example of the solvent includes a ketone solvent such as acetone, methyl ethyl ketone, methyl isobutyl ketone and cyclohexanone; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; a nitrile solvent such as acetonitrile; and a halogenated hydrocarbon solvent such as dichloromethane and chloroform.

The temperature for the reaction of the carbonyl halide and the alcohol compound is not particularly restricted and may be appropriately adjusted. For example, the temperature may be adjusted to 0°C or higher and 250°C or lower. The temperature is more preferably 10°C or higher, even more preferably 20°C or higher, and more preferably 200°C or lower or 150°C or lower, even more preferably 100°C or lower or 80°C or lower. When the base is not used or the base is used for further accelerating the reaction, the temperature may be adjusted to be relatively high. For example, the temperature may be adjusted to 50°C or higher or 100°C or higher.

The time to react the carbonyl halide and the alcohol compound is not particularly restricted and may be appropriately adjusted. For example, the time is preferably 0.5 hours or more and 50 hours or less. The reaction time is more preferably 1 hour or more, even more preferably 5 hours or more, and more preferably 30 hours or less, even more preferably 20 hours or less. For example, even after the production of the carbonyl halide is completed, the reaction mixture may be continuously stirred until the consumption of the alcohol compound is confirmed.

When the monovalent alcohol compound (I) is used, the chain carbonate compound represented by the following formula (III) is produced by the reaction of the carbonyl halide and the alcohol compound. When the divalent alcohol compound (II) is used, the polycarbonate compound comprising the unit represented by the following formula (IV-1) or the cyclic carbonate compound represented by the following formula (IV-2) is produced. When the divalent alcohol compound (II) is used, whether the polycarbonate compound (IV-1) is produced or the cyclic carbonate compound (IV-2) is produced and the production ratio thereof are mainly dependent on the distance between two hydroxy groups and the flexibility of the chemical structure of the divalent alcohol compound (II) and may be specifically determined by a preliminary experiment or the like.

R¹-O-C(=O)-O-R¹ (III)

[-O-R²-O-C(=O)-] (IV-1)

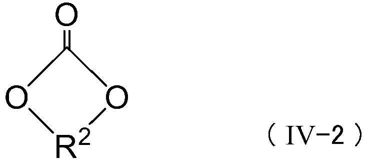

The polymerization reaction may proceed very efficiently by the present invention to produce a polycarbonate compound having a large molecular weight. For example, the weight-average molecular weight in polystyrene equivalents measured by gel permeation chromatography (GPC) of the polycarbonate compound produced by the present invention method is preferably 10,000 or more and 1,000,000 or less, and the number average molecular weight thereof is preferably 5,000 or more and 500,000 or less.

### · Post-reaction step - Production of halogenated formate ester

A halogenated formate ester can be mainly produced by adjusting the usage amount of the alcohol compound to relatively small without using a base in the above-described method for producing a carbonate compound. For example, the molar ratio of the alcohol compound to methane may be adjusted to less than 1. The molar ratio is preferably 0.9 or less and more preferably 0.8 or less. Both of a carbonate compound and a halogenated formate ester may be produced depending on the conditions. The above-described monovalent alcohol compound (I) can be used as an alcohol compound. A halogenated formate fluorinated ester can be produced from the monovalent fluorinated alcohol compound (I), and a halogenated formate non-fluorinated ester can be produced from the monovalent non-fluorinated alcohol compound (I).

### · Post-reaction step - Production of isocyanate compound

An isocyanate compound can be produced by reacting the carbonyl halide and a primary amine compound. An isocyanate compound is useful as a raw material of a carbamate compound, a urethane compound or the like. A primary amine compound may be used in place of the alcohol compound in the above-described method for producing a carbonate compound except for the following points as the reaction embodiment.

A primary amine compound is not particularly restricted as long as the compound has 1 or more primary amino groups (-NH₂ groups). An example of the primary amine compound includes the primary amine compound (V) : R³-(NH₂)ₘ wherein R³ is an m-valent organic group, and m is an integer of 1 or more and 6 or less, preferably 5 or less, 4 or less or 3 or less, more preferably 1 or 2, and even more preferably 2.

An example of the monovalent organic group among the organic group R³ includes the group corresponding to the monovalent organic group R¹ in the above-described method for producing a carbonate compound, and an example of the divalent organic group includes the group corresponding to the divalent organic group R². An example of the 3 or more-valent organic group includes the 3 or more-valent organic group derived from the examples of the monovalent organic group R¹. For example, the trivalent organic group derived from the C₁₋₁₀ alkyl group, the C₂₋₁₀ alkenyl group and the C₂₋₁₀ alkynyl group as the monovalent organic group is a C₁₋₁₀ alkane-triyl group, a C₂₋₁₀ alkene-triyl group and a C₂₋₁₀ alkyne-triyl group.

The isocyanate compound (VI): R³-(N=C=O)ₘ can be produced by reacting the carbonyl halide and the primary amine compound (V). The produced R³-(N=C=O)ₘ may be possibly reacted with the primary amine compound (V) to produce a urea compound: R³-[NH-C(=O)-NH-R³]ₘ, and it is preferred to inhibit such a reaction that the usage amount of the primary amine compound (V) is adjusted to relatively small, a salt of the primary amine compound (V) is used, or a base is not used. For example, the molar ratio of the primary amine compound (V) to methane is adjusted to 1 or less. In addition, an isocyanate compound can be efficiently produced by the following conditions: the produced carbonyl halide is dissolved in a solvent to prepare a carbonyl halide solution, and the primary amine compound (V) or a solution thereof is added to the carbonyl halide solution while the molar ratio of the carbonyl halide to the primary amine compound (V) is maintained at more than 1.

When the target compound is an isocyanate compound, the molar ratio of the primary amine compound (V) to the produced carbonyl halide is preferably adjusted to 1 or less. Since it may be difficult in some cases to predict the accurate amount of the produced carbonyl halide, the molar ratio of the primary amine compound (V) to the used methane is preferably adjusted to less than 1. The molar ratio is preferably 0.5 or less, more preferably 0.2 or less, and preferably 0.001 or more, more preferably 0.05 or more.

When the target compound is an isocyanate compound, a salt of the primary amine compound (V) is preferably used, since an isocyanate compound is hardly reacted with an amine salt. An example of such a salt includes an inorganic acid salt such as hydrochloride salt, hydrobromide salt, hydroiodide salt, sulfate salt, nitrate salt, perchlorate salt and phosphate salt; and an organic salt such as oxalate salt, malonate salt, maleate salt, fumarate salt, lactate salt, malate salt, citrate salt, tartrate salt, benzoate salt, trifluoroacetate salt, acetate salt, methanesulfonate salt, p-toluenesulfonate salt and trifluoromethanesulfonate salt.

The temperature for the reaction of the carbonyl halide and the primary amine compound is preferably adjusted to be lower than the temperature for the reaction with the alcohol compound in order to maintain the liquid state of the carbonyl halide. For example, the reaction temperature may be adjusted to 15°C or lower, and is preferably 10°C or lower, more preferably 5°C or lower and even more preferably 2°C or lower. The lower limit of the temperature is not particularly restricted, and the temperature is preferably -80°C or higher and more preferably -20°C or higher or -15°C or higher.

When the target compound is an isocyanate compound and a base is used, the base is preferably 1 or more bases selected from a heterocyclic aromatic amine and a non-nucleophilic strong base. The heterocyclic aromatic amine means a compound that contains at least one hetero ring and at least one amine functional group other than -NH₂. An example of the heterocyclic aromatic amine includes pyridine and a derivative thereof, such as pyridine, α-picoline, β-picoline, γ-picoline, 2,3-lutidine, 2,4-lutidine, 2,6-lutidine, 3,5-lutidine, 2-chloropyridine, 3-chloropyridine, 4-chloropyridine, 2,4,6-trimethylpyridine and 4-dimethylaminopyridine.

The "non-nucleophilic organic base" means a base in which the nucleophilicity of the lone electron pair on the nitrogen atom is weak due to a steric hindrance but of which basicity is strong. An example thereof includes triethylamine, N,N-diisopropylethylamine, tripropylamine, triisopropylamine, tributylamine, tripentylamine, trihexylamine, triheptylamine, trioctylamine, tridecylamine, tridodecylamine, triphenylamine, tribenzylamine, N,N-diisopropylethylamine, 1,5,7-triazabicyclo[4.4.0]dec-5-ene (TBD), 7-methyl-1,5,7-triazabicyclo[4.4.0]dec-5-ene (MTBD), 1,8-diazabicyclo[5.4.0]undec-7-ene (DBU), 1,5-diazabicyclo[4.3.0]non-5-ene (DBN) and 1,1,3,3-tetramethylguanidine (TMG). In addition, a base of which basicity is relatively high may be used. For example, TBD (pK_{BH+}: 25.98), MTBD (pK_{BH+}: 25.44), DBU (pK_{BH+}: 24.33), DBN (pK_{BH+}: 23.89) and TMG (pK_{BH+}: 23.30) can be used as a base of which basicity (pK_{BH+}) in acetonitrile is 20 or more.

In addition, a versatile organic amine such as trimethylamine, dimethylethylamine, diethylmethylamine, N-ethyl-N-methylbutylamine and 1-methylpyrrolidine can be used as the base.

### · Post-reaction step - Production of carbamoyl halide compound

A carbamoyl halide compound can be produced by reacting the carbonyl halide with a secondary amine compound. A carbamoyl halide compound is useful as a synthetic intermediate of a physiologically active substance such as medicine and agricultural chemicals. An example of such a physiologically active substance includes a carbamate ester, such as a carbamate compound having an insecticidal property. A carbamoyl halide compound having 2 or more of carbamoyl halide groups [-N(C=O)-Cl] can be used as a raw material of polyurea and polyurethane. A secondary amine compound may be used in place of a primary amine compound in the above-described method for producing an isocyanate compound as the reaction embodiment.

A secondary amine compound is not particularly restricted as long as the compound has 1 or more secondary amino groups (-NHR groups). For example, the secondary amine compound (XI): R²¹-(NH-R²²)ₘ can be used. In the formula, R²¹ is an m-valent organic group; m is an integer of 1 or more and 6 or less, and is preferably 5 or less, 4 or less or 3 or less, more preferably 1 or 2, and even more preferably 2; and R²² is a monovalent organic group. An example of R²² includes a group similar to the monovalent organic group R¹ in the above-described method for producing a carbonate compound, and R²² is preferably a C₁₋₁₀ alkyl group.

The carbamoyl halide compound (XII): R²¹-[N(R²²)-C(=O)-X]ₘ wherein X is 1 or more halogeno groups selected from the group consisting of chloro, bromo and iodo can be produced by reacting the carbonyl halide and the secondary amine compound (XI). The produced R²¹-[N(R²²)-C(=O)-X]ₘ may be possibly reacted with the secondary amine compound (XI) to produce a urea compound. It is preferred to inhibit the reaction that the usage amount of the secondary amine compound (XI) is adjusted to relatively small or a base is not used. For example, the molar ratio of the secondary amine compound (XI) to methane is adjusted to 1 or less. In addition, a carbamoyl halide compound can be efficiently produced by the following conditions: the produced carbonyl halide is dissolved in a solvent to prepare a carbonyl halide solution, and the secondary amine compound (XI) or a solution thereof is added to the carbonyl halide solution while the molar ratio of the carbonyl halide to the secondary amine compound (XI) is maintained at more than 1.

When the target compound is a carbamoyl halide compound, the usage amount of the secondary amine compound (XI) is preferably adjusted to relatively small. For example, the molar ratio of the secondary amine compound (XI) to the produced carbonyl halide is preferably adjusted to 1 or less. Since it may be difficult in some cases to predict the accurate amount of the produced carbonyl halide, the molar ratio of the secondary amine compound (XI) to the used methane is preferably adjusted to less than 1. The molar ratio is preferably 0.5 or less, more preferably 0.2 or less, and preferably 0.001 or more, more preferably 0.05 or more. On the one hand, the target compound is a urea compound, the molar ratio is preferably 2 or more, preferably 4 or more, and preferably 20 or less, more preferably 15 or less.

The temperature for the reaction of the carbonyl halide and the secondary amine compound (XI) is preferably adjusted to be lower than the temperature for the reaction with the alcohol compound in order to maintain the liquid state of the carbonyl halide. For example, the temperature may be adjusted to 15°C or lower, and is preferably 10°C or lower, more preferably 5°C or lower and even more preferably 2°C or lower. The lower limit of the temperature is not particularly restricted, and the temperature is preferably -80°C or higher and more preferably -20°C or higher or -15°C or higher.

When the target compound is a carbamoyl halide compound and a base is used, 1 or more bases selected from a heterocyclic aromatic amine and a non-nucleophilic strong base exemplified in the above-described conditions of the method for producing an isocyanate compound is preferably used as the base. When the target compound is a urea compound, the molar ratio of the secondary amine compound to methane or the produced carbonyl halide is preferably adjusted to more than 1. The molar ratio is preferably 1.5 or more and more preferably 2 or more.

### · Post-reaction step - Production of NCA

An amino acid-N-carboxylic anhydride (VIII) (NCA) can be produced by using the amino acid compound (VII) in place of the alcohol compound in the above-described method for producing a carbonate compound. 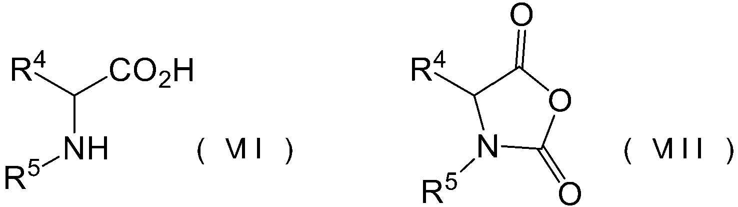 wherein
R⁴ is an amino acid side chain group wherein a reactive group is protected,
R⁵ is H or P¹-[-NH-CHR⁶-C(=O)-]ₗ- wherein R⁶ is an amino acid side chain group wherein a reactive group is protected, P¹ is a protective group of the amino group, 1 is an integer of 1 or more, and when l is an integer of 2 or more, a plurality of R⁶ may be the same as or different from each other.

### · Post-reaction step - Production of Vilsmeier reagent

A Vilsmeier reagent (X) can be produced by reacting the carbonyl halide and the amide compound (IX). A Vilsmeier reagent can be produced similarly to the above-described method for producing a carbonate compound except that the amide compound (IX) is used in place of the alcohol compound and a base is not used. 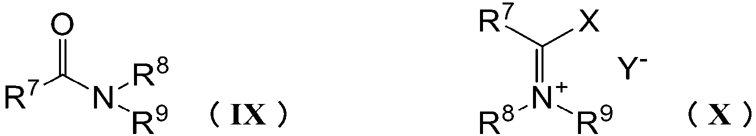 wherein
R⁷ is a hydrogen atom, a C₁₋₆ alkyl group or an optionally substituted C₆₋₁₂ aromatic hydrocarbon group,
R⁸ and R⁹ are independently a C₁₋₆ alkyl group or an optionally substituted C₆₋₁₂ aromatic hydrocarbon group, or R⁸ and R⁹ may form a 4 or more and 7 or less-membered ring structure together with each other,
X is a halogeno group selected from the group consisting of chloro, bromo and iodo,
Y⁻ is a counter anion.

The substituent group that the C₆₋₁₂ aromatic hydrocarbon group may optionally have is not particularly restricted as long as the substituent group does not inhibit the reaction of the present invention. An example thereof includes 1 or more substituent groups selected from the group consisting of a C₁₋₆ alkyl group, a C₁₋₆ alkoxy group, a halogeno group, a nitro group and a cyano group. The number of the substituent group is not particularly restricted as long as the substitution is possible and may be 1 or more and 5 or less. The number is preferably 3 or less, more preferably 2 or less, and even more preferably 1. When the substituent group number is 2 or more, the substituent groups may be the same as or different from each other.

An example of the 4 or more and 7 or less-membered ring structure that is formed by R⁸, R⁹ and the nitrogen atom together with each other includes a pyrrolidyl group, a piperidyl group and a morpholino group.

An example of the specific amide compound (IX) includes N,N-dimethylformamide (DMF), N,N-dimethylacetamide (DMA), N-methyl-N-phenylformamide, N-methylpyrrolidone (NMP), 1,3-dimethylimidazolidinone (DMI), tetramethylurea, tetraethylurea and tetrabutylurea, and DMF is preferred in terms of versatility and a cost.

The Y⁻ in the formula (X) is not particularly restricted, and an example thereof includes a chloride ion, a bromide ion and an iodide ion derived from the halogen molecule gas.

The usage amount of the amide compound may be appropriately adjusted as long as the reaction proceeds successfully, and the usage amount to 1 mole of the methane may be adjusted to 0.1 mole or more and 100 mole or less.

When the carbonyl halide and the amide compound are reacted, a solvent may be used. For example, a solvent is mixed in a composition containing the amide compound. An example of the solvent includes a ketone solvent such as acetone, methyl ethyl ketone, methyl isobutyl ketone and cyclohexanone; an ester solvent such as ethyl acetate; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; and a nitrile solvent such as acetonitrile.

The temperature for the reaction of the carbonyl halide and the amide compound is not particularly restricted and may be appropriately adjusted. For example, the temperature may be adjusted to 0°C or higher and 120°C or lower. The temperature is more preferably 10°C or higher, even more preferably 20°C or higher, and more preferably 100°C or lower, even more preferably 80°C or lower or 50°C or lower.

The time to react the carbonyl halide and the amide compound is not particularly restricted and may be appropriately adjusted. For example, the time is preferably 0.5 hours or more and 50 hours or less. The reaction time is more preferably 1 hour or more, even more preferably 5 hours or more, and more preferably 30 hours or less, even more preferably 20 hours or less. For example, even after the production of the carbonyl halide is completed, the reaction mixture may be continuously stirred until the consumption of the amide compound is confirmed.

A Vilsmeier-Haack reaction using a Vilsmeier reagent allows the formylation or ketonization of an aromatic compound having an active group. In addition, it has been known that the carboxy group of a carboxylic acid compound can be transformed to a haloformyl group by a Vilsmeier reagent. Further, a formate ester can be produced by reacting a Vilsmeier reagent and a hydroxy group-containing compound. The inventor of the present invention has found that when DMF and a carbamoyl compound having a carbamoyl group (-C(=O)-NH₂) coexist, a Vilsmeier reagent may act as a dehydrating agent and thus a nitrile compound having a cyano group can be produced. An example of a carbamoyl compound includes R¹-C(=O)-NH₂, and an example of a nitrile compound includes R¹-CN.

An aromatic compound having an active group means an aromatic compound activated by a substituent group or the like. The aromatic compound is hereinafter described as an "active aromatic compound". For example, a hydroxy group and an amino group such as an alkylamino group substituted by an alkyl group strongly activate an aromatic compound. An alkylcarbonylamino group (-N(C=O)R), an alkylcarbonyloxy group (-O(C=O)R), an ether group (-OR), an alkyl group (-R) wherein R is an alkyl group and is preferably a C₁₋₆ alkyl group and an aromatic group activate an aromatic compound. The substituent groups are hereinafter referred to as an activating group. In addition, a compound that is constructed by condensing aromatic rings and of which conjugated system is extended, such as anthracene, is also activated and can be subjected to formylation or ketonization by a Vilsmeier reagent. The pi electron at the activated part may be electrophilically reacted with a Vilsmeier reagent to be subjected to formylation or ketonization.

The active aromatic compound is not particularly restricted as long as the active aromatic compound is activated and can be subjected to formylation or ketonization by a Vilsmeier reagent. An example thereof includes a C₆₋₁₂ aromatic hydrocarbon, such as benzene and naphthalene, that is substituted with the above-described activating group; a condensed aromatic hydrocarbon, such as phenanthrene and anthracene, that may be substituted with the above-described activating group; a 5-membered heteroaryl group, such as pyrrol, imidazole, pyrazole, thiophen, furan, oxazole, isoxazole, thiazole, isothiazole and thiadiazole, that may be substituted with the above-described activating group; a 6-membered heteroaryl, such as pyridine, pyrazine, pyrimidine and pyridazine, that may be substituted with the above-described activating group; a condensed heteroaryl, such as indole, isoindole, quinoline, isoquinoline, benzofuran, isobenzofuran and chromene, that may be substituted with the above-described activating group.

An activating group-containing aromatic compound, a carboxylic acid compound and a hydroxy group-containing compound as a substrate compound of the above-described reaction may be added to the reaction mixture after the carbonyl halide-containing gas is blown into the composition containing the amide compound, or added to the reaction mixture before or while the carbonyl halide-containing gas is blown into the composition containing the amide compound.

The usage amount of the activating group-containing aromatic compound, the carboxylic acid compound and the hydroxy group-containing compound may be appropriately adjusted. For example, the usage amount may be adjusted to 0.1 times or more by mole and 1.0 time or less by mole to the amide compound.

A Vilsmeier reagent is also useful for producing a carboxylic acid halide from a carboxylic acid compound. A Vilsmeier reagent reverts to an amide compound after the Vilsmeier reagent halogenates a carboxylic acid compound. An ester compound can be produced by reacting the produced carboxylic acid halide with an alcohol compound, and a carboxylic anhydride can be produced by reacting the produced carboxylic acid halide with a carboxylic acid. When a carboxylic acid compound and a base are used in place of the amide compound, the carboxylic acid compound may be anionized by the base and then the anionized carboxylate compound may be directly transformed to a carboxylic acid halide by the carbonyl halide. Such a carboxylic acid halide can be also used for producing an ester compound and a carboxylic anhydride.

### · Post-reaction step - Production of urea compound

A urea compound can be produced by reacting the carbonyl halide with a primary amine compound or a secondary amine compound. Specifically, a urea compound can be mainly produced by adjusting the usage amount of a primary amine compound or a secondary amine compound to relatively large in the above-described method for producing an isocyanate compound or method for producing a carbamoyl halide compound. For example, the molar ratio of a primary amine compound or a secondary amine compound to methane is adjusted to 1 or more. An isocyanate compound or a carbamoyl halide compound may be produced by reacting the carbonyl halide with a primary amine compound or a secondary amine compound, and further a urea compound may be produced by reacting the isocyanate compound or the carbamoyl halide compound with the primary amine compound or the secondary amine compound. The molar ratio is preferably 1.5 or more, more preferably 2 or more or 4 or more, and preferably 20 or less, more preferably 15 or less.

The primary amine compound (V): R³-(NH₂)ₘ exemplified in the above-described method for producing an isocyanate compound can be used as a primary amine compound. The secondary amine compound (XI): R²¹-(NH-R²²)ₘ exemplified in the above-described method for producing a carbamoyl halide compound can be used as a secondary amine compound.

When a primary amine compound or a secondary amine compound is used in this reaction step, a base is preferably used, since a hydrogen halide produced as a side product may form a salt to inhibit the reaction. An example of the base includes 1 or more bases selected from a heterocyclic aromatic amine and a non-nucleophilic strong base. The usage amount of the base may be appropriately adjusted in the range that the reaction proceeds successfully. For example, the usage amount to 1 mole of methane may be adjusted to 1 mole or more and 10 mole or less.

An isocyanate compound or a carbamoyl halide compound can be produced by the reaction of the carbonyl halide with the primary amine compound (V) or the secondary amine compound (XI). Further, a urea compound is produced by the reaction of the produced isocyanate compound or carbamoyl halide compound with the primary amine compound (V) or the secondary amine compound (XI).

### · Post-treatment step

Since many carbonyl halides are toxic, it is preferred that the produced carbonyl halide is not leaked out of the reaction system. For example, it is preferred that the gas phase discharged from the reaction vessel for the reaction of the produced carbonyl halide is supplied into an alcohol trap, and the gas phase discharged from the alcohol trap is further supplied into an alkali trap. The alcohol trap may be cooled as long as the alcohol to be used is not coagulated. For example, the alcohol trap may be cooled to -80°C or higher and 50°C or lower. In addition, for example, a sodium hydroxide aqueous solution and a saturated sodium hydrogencarbonate aqueous solution may be used for the alkali trap.

When the compound produced from the carbonyl halide is a relatively unstable compound such as an isocyanate compound, further a reactive substrate compound may be added to the reaction mixture after the carbonyl halide is reacted. When the compound produced from the carbonyl halide is a relatively stable compound such as a carbonate compound, the target compound may be purified from the reaction mixture. For example, water and a water-insoluble organic solvent such as chloroform are added to the reaction mixture, the aqueous phase and the organic phase are separated, the organic phase is dried over anhydrous sodium sulfate or anhydrous magnesium sulfate and then concentrated under reduced pressure, and the target compound may be purified by chromatography or the like.

The present application claims the benefit of the priority date of Japanese patent application No. 2023-176533 filed on October 12, 2023. All of the contents of the Japanese patent application No. 2023-176533 filed on October 12, 2023, are incorporated by reference herein.

### EXAMPLES

Hereinafter, the examples are described to demonstrate the present invention more specifically, but the present invention is in no way restricted by the examples, and the examples can be appropriately modified to be carried out within a range that adapts to the contents of this specification. Such a modified example is also included in the range of the present invention.

### Example 1: Synthesis of carbonyl chloride from highly pure methane gas

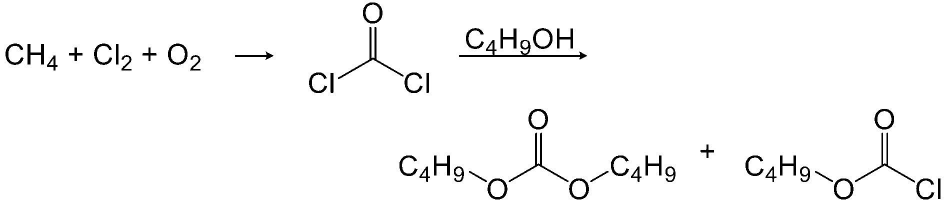

The reaction system schematically demonstrated in Figure 1 was constructed, and the following reagents and devices were used.
Methane : manufactured by Sumitomo Seika Chemicals, purity: >99.0%
Calcium hypochlorite: manufactured by NACALAI TESQUE, effective chlorine: >60%
Hydrochloric acid: manufactured by NACALAI TESQUE, concentration: 35%
1-Butanol: manufactured by FUJIFILM Wako Pure Chemical, purity: >99.0%
Calcium chloride: manufactured by FUJIFILM Wako Pure Chemical, purity: >95.0%
Air pump: "Nisso Air Pump Silent β-60" manufactured by Marukan
Mass flow controller: "MODEL 8500MC" manufactured by KOFLOC
Ultrasonic washer: "1510J-MT" manufactured by BRANSONIC
Hot plate: "C-MAG HP7" manufactured by IKA

Calcium hypochlorite (11.4 g, 80 mmol) was added into a three-necked flask immersed in a water bath, and chlorine gas was continuously produced in accordance with the following formula by adding 11.3 mol/L hydrochloric acid using a syringe pump at the injection rate of 228.1 µL/min under atmospheric temperature while ultrasound of 70 W and 42 kHz was irradiated using the ultrasonic washer.

Ca(ClO)₂·3H₂O + 4HCl → CaCl₂ + 2Cl₂ + 5H₂O

Air to be dried was supplied into a gas drying tube having the inner diameter of 16 mm and the length of 265 mm filled with silica gel using the air pump. The flow rate of the dried air was adjusted as shown in Table 1 using the mass flow controller, and the dried gas was supplied into the above three-necked flask. The obtained mixed gas of chlorine and air was dried by supplying the gas into a gas drying tube having the inner diameter of 16 mm and the length of 265 mm filled with calcium chloride and joined together with methane gas of which flow rate was controlled using the mass flow controller and that was supplied from a cylinder at a T-shaped mixer to prepare a mixed gas of chlorine/air/methane. The mixed gas was supplied into a PTFE coil heater and a flow photoreactor. The flow photoreactor was composed of a square borosilicate glass vessel (outer size: 220 mm × 185 mm × 30 mm, thickness: 3.3 mm, inner volume: 891 cm³) or a cylindrical borosilicate glass vessel (outer size: φ55 mm × 150 mm, thickness: 2.0 mm, inner volume: 300 cm³), 365 nm LED lamp (manufactured by Polarstar, peak wavelength: 365 nm, 45 W, LED (14 cm × 18 cm), illumination intensity: 36.5 mW/cm²) or 405 nm LED lamp (manufactured by Polarstar, peak wavelength: 405 nm, 30 W, LED (14 cm × 18 cm), illumination intensity: 57.2 to 54.5 mW/cm²) and a hot plate. The residence time of the reaction gas in the flow photoreactor was controlled by adjusting the flow rates of air and methane gas and the production rate of chlorine.

The gaseous product produced by the system was blown into 1-butanol in the first reaction vessel to produce the mixed product of chloroformate ester and carbonate. The gas derived from the first reaction vessel was similarly supplied to the second reaction vessel containing 1-butanol. The reaction mixtures of the first reaction vessel and the second reaction vessel were analyzed by ¹H NMR to determine the amounts of the produced butyl chloroformate and dibutyl carbonate and calculate the amount of the produced carbonyl chloride. The experimental conditions are shown in Table 1, and the experimental results are shown in Table 2.

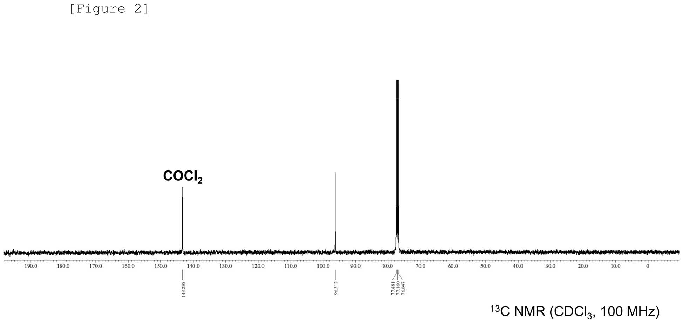

In addition, the reaction gas derived from the photoreaction vessel was blown into CDCl₃ at 0°C to be analyzed by ¹³C NMR. The result is shown in Figure 2.

**Table 1**

| Experiment number | Flow rate | | Photoreaction vessel volume | CH₄ total amount | Cl₂ total amount | Residence time | Light source | Coil heater temp. | Photoreaction vessel temperature |
|---|---|---|---|---|---|---|---|---|---|
| | CH₄ | Air (O₂: mmol/min) | | | | | | | |
| 1 | 14.9mL/min (0.62mmol/min) | 30.0mL/min (0.25mmol/min) | 891mL | 56 mmol | | 14.8min | 365nm 45W | 150°C | 100°C |
| 2 | | | | | | | | r.t. | r.t. |
| 3 | | | | | | | | | 0°C |
| 4 | | | 300mL | | 56 mmol | 5.9min | | | r.t. |
| 5 | | | 891mL | | | 14.8min | 405nm 30W | | |
| 6 | 9.0mL/min (0.40mmol/min) | 45.0mL/min (0.38mmol/min) | | 36 mmol | | 9.2min | 365nm 45W | | |
| 7 | | | | | | 8.4min | | | 50°C |

**Table 2**

| Experiment number | A: chloroformate ester | B: carbonate | Carbonyl chloride amount (A + B) | Carbonyl chloride conversion rate |
|---|---|---|---|---|
| 1 | 24mmol | trace | 24mmol | 42% |
| 2 | 26mmol | trace | 26mmol | 47% |
| 3 | 24mmol | trace | 24mmol | 42% |
| 4 | 21mmol | trace | 21mmol | 38% |
| 5 | 22mmol | trace | 22mmol | 39% |
| 6 | 17mmol | trace | 17mmol | 46% |
| 7 | 19mmol | trace | 19mmol | 53% |

Carbonyl chloride was confirmed in the gas produced by reacting methane, chlorine and oxygen as shown in Figure 2. The other peaks are the peaks of CDCl₃ as a solvent.

Even when the temperature of the photoreaction vessel was increased from room temperature (Experiment number 2) to 100°C (Experiment number 1), and even when the photoreaction vessel was cooled to 0°C (Experiment number 3), the amounts of the produced chloroformate ester and carbonate were largely unchanged as the results of Experiment numbers 1 to 3. Thus, the amount of the produced carbonyl chloride might be largely unchanged.

When a relatively small photoreaction vessel was used (Experiment number 4), the yield of chloroformate ester was slightly decreased but was not decreased significantly. This result is considered to be due to the shortened residence time of the reaction gas. The amount of the produced carbonyl chloride might be largely unchanged on the basis of the result.

Even when the irradiation light was changed to low energy light having relatively long wavelength (Experiment number 5), the yield of chloroformate ester was slightly decreased but was not decreased significantly. This result may indicate that the amount of the produced carbonyl chloride was largely unchanged.

When the amount of the air supplied to highly pure methane gas was increased to reduce the methane concentration in the reaction gas (Experiment numbers 6 and 7), the conversion rate from methane to carbonyl chloride tended to be higher.

### Example 2: Synthesis of carbonyl chloride from city gas

The reaction system schematically shown in Figure 1 was used.

Calcium hypochlorite (18.5 g, 130 mmol) was added into a three-necked flask immersed in an ice bath, and chlorine gas was continuously produced by adding 2.1 mmol/L hydrochloric acid using a syringe pump at the injection rate of 370 µL/min under atmospheric temperature while ultrasound of 70 W and 42 kHz was irradiated using the ultrasonic washer.

Air to be dried was supplied into a gas drying tube having the inner diameter of 16 mm and the length of 265 mm filled with silica gel using the air pump. The flow rate of the dried air was adjusted to 55 mL/min (O₂ flow rate: 0.46 mmol/min) using the mass flow controller, and the dried gas was supplied into the above three-necked flask. City gas of which flow rate was adjusted to 13.2 mL/min (methane flow rate: 0.55 mmol/min) using the mass flow controller was joined together with the obtained mixed gas of chlorine and air to be supplied into the PTFE coil heater to prepare a mixed gas of chlorine/air/city gas. The mixed gas was supplied into the flow photoreactor. The city gas was provided from OSAKA GAS and composed of methane: about 88.9 vol%, ethane: about 6.8 vol%, propane: about 3.1 vol% and butane: about 1.2 vol%. The flow photoreactor was composed of a square borosilicate glass vessel (outer size: 220 mm × 185 mm × 30 mm, thickness: 3.3 mm, inner volume: 891 cm³) and 365 nm LED lamp (manufactured by Polarstar, peak wavelength: 365 nm, 45 W, LED (14 cm × 18 cm), illumination intensity: 54.7 to 54.4 mW/cm²).

The reaction time was adjusted to 2 hours. The amounts of the methane and chlorine supplied into the flow photoreactor during the reaction were 50 mmol and 56 mmol. The residence time of the reaction gas in the flow photoreactor was 9 minutes. The temperatures of the flow photoreactor and the coil heater were not controlled during the reaction.

The gaseous product produced by the system was blown into 1-butanol in the first reaction vessel to produce the mixed product of chloroformate ester and carbonate. The gas derived from the first reaction vessel was similarly supplied to the second reaction vessel containing 1-butanol. The reaction mixtures of the first reaction vessel and the second reaction vessel were analyzed by ¹H NMR, and the amount of the produced carbonyl chloride was determined by the amounts of the produced ethyl chloroformate and dibutyl carbonate. The result is shown in Table 3.

**Table 3**

| A: chloroformate ester | B: carbonate | Amount of carbonyl chloride (A + B) | Carbonyl chloride conversion rate |
|---|---|---|---|
| 16mmol | - | 16mmol | 32% |

Carbonyl chloride could be produced from methane that contained other hydrocarbon gas such as ethane as the result shown in Table 3.

Since the conversion rate from methane to carbonyl chloride was not significantly different from that of Example 1, the other hydrocarbon gas such as ethane might be decomposed into the compound other than carbonyl chloride.

### Example 3: Synthesis of carbonyl chloride from biogas

The reaction system schematically shown in Figure 1 was used.

Calcium hypochlorite was added into a three-necked flask immersed in an ice bath. Chlorine gas was continuously produced by adding 2.1 mmol/L hydrochloric acid using a syringe pump at the injection rate of 370 µL/min under atmospheric temperature while ultrasound of 70 W and 42 kHz was irradiated using the ultrasonic washer. The amount of calcium hypochlorite was adjusted to 18.5 g (130 mmol) in Experiment numbers 1 and 2, and the amount was adjusted to 129.5 g (906 mmol) in Experiment number 3 in which the reaction time was adjusted to longer.

Air to be dried was supplied into a gas drying tube having the inner diameter of 16 mm and the length of 265 mm filled with silica gel using the air pump. The flow rate of the dried air was adjusted to 55 mL/min (O₂ flow rate: 0.46 mmol/min) using the mass flow controller, and the dried gas was supplied into the above three-necked flask. The obtained mixed gas of chlorine and air was joined together with biogas ("TLF-06472" manufactured by AIR WATER) of which flow rate was adjusted to 24.8 mL/min (methane flow rate: 0.55 mmol/min) using the mass flow controller to be supplied into the PTFE coil heater to prepare a mixed gas of chlorine/air/biogas. The mixed gas was supplied into the flow photoreactor. The used biogas was composed of methane: 53 vol%, carbon dioxide: 34 vol%, nitrogen: 8.3 vol% and oxygen: 2.5 vol%. The flow photoreactor was composed of a square borosilicate glass vessel (outer size: 220 mm × 185 mm × 30 mm, thickness: 3.3 mm, inner volume: 891 cm³) and 365 nm LED lamp (manufactured by Polarstar, peak wavelength: 365 nm, 45 W, LED (14 cm × 18 cm), illumination intensity: 54.7 to 54.4 mW/cm²) or 405 nm LED lamp (manufactured by Polarstar, peak wavelength: 405 nm, 30 W, LED (14 cm × 18 cm), illumination intensity: 57.2 to 54.5 mW/cm²).

The residence time of the reaction gas in the flow photoreactor was 11.2 minutes. The temperatures of the flow photoreactor and the coil heater were not controlled during the reaction.

The gaseous product produced by the system was blown into 1-butanol in the first reaction vessel to produce the mixed product of chloroformate ester and carbonate. The gas derived from the first reaction vessel was similarly supplied to the second reaction vessel containing 1-butanol. The reaction mixtures of the first reaction vessel and the second reaction vessel were analyzed by ¹H NMR, and the amount of the produced carbonyl chloride was determined by the amounts of the produced ethyl chloroformate and dibutyl carbonate. The experimental conditions are shown in Table 4, and the experimental results are shown in Table 5.

**Table 4**

| Experiment number | CH₄ total amount | Cl₂ total amount | Light source | Reaction time |
|---|---|---|---|---|
| 1 | 50mmol | 95mmol | 365nm, 45W | 2h |
| 2 | | | 405nm, 30W | |
| 3 | 347mmol | 665mmol | 365nm, 45W | 10.5h |

**Table 5**

| Experiment number | A: chloroformate ester | B: carbonate | Carbonyl chloride amount (A + B) | Carbonyl chloride conversion rate |
|---|---|---|---|---|
| 1 | 50mmol | - | 50mmol | >99% |
| 2 | 46mmol | - | 46mmol | 92% |
| 3 | 326mmol | - | 326mmol | 94% |

Carbonyl chloride could be produced from biogas with high conversion rate as the result shown in Table 5.

The reason may be that the excessive chlorination reaction and the excessive oxidation reaction of methane were suppressed, since biogas contains relatively large amount of an inert gas such as carbon dioxide and nitrogen and thus the concentration of methane is relatively low.

### Example 4: Synthesis of carbonyl chloride from biogas

The reaction system schematically shown in Figure 1 was used, and the experiment was carried out on a larger scale than the above-described Example 3.

Calcium hypochlorite (39.0 g, 273 mmol) was added into a three-necked flask immersed in an ice bath. Chlorine gas was continuously produced by adding 2.1 mmol/L hydrochloric acid using a syringe pump at the injection rate of 1029 µL/min under atmospheric temperature while ultrasound of 70 W and 42 kHz was irradiated using the ultrasonic washer.

Air to be dried was supplied into a gas drying tube having the inner diameter of 16 mm and the length of 265 mm filled with silica gel using the air pump. The flow rate of the dried air was adjusted to the flow rate shown in Table 6 using the mass flow controller, and the dried gas was supplied into the above three-necked flask. The obtained mixed gas of chlorine and air was joined together with biogas ("TLF-06472" manufactured by AIR WATER) of which flow rate was adjusted using the mass flow controller to be supplied into the PTFE coil heater to prepare a mixed gas of chlorine/air/biogas. The mixed gas was supplied into the flow photoreactor. The used biogas was composed of methane: 53 vol%, carbon dioxide: 34 vol%, nitrogen: 8.3 vol% and oxygen: 2.5 vol%. The flow photoreactor was composed of a square borosilicate glass vessel (outer size: 220 mm × 185 mm × 50 mm, thickness: 3.3 mm, inner volume: 1652 cm³) or serially concatenated two square borosilicate glass vessels (outer size: 220 mm × 90 mm × 50 mm, thickness: 3.3 mm, inner volume: 772 cm³) and 365 nm LED lamp (manufactured by Polarstar, peak wavelength: 365 nm, 45 W, LED (14 cm × 18 cm), illumination intensity: 54.7 to 54.4 mW/cm²).

The temperatures of the flow photoreactor and the coil heater were not controlled during the reaction.

The gaseous product produced by the system was blown into 1-butanol in the first reaction vessel to produce the mixed product of chloroformate ester and carbonate. The gas derived from the first reaction vessel was similarly supplied to the second reaction vessel containing 1-butanol. The reaction mixtures of the first reaction vessel and the second reaction vessel were analyzed by ¹H NMR, and the amount of the produced carbonyl chloride was determined by the amounts of the produced ethyl chloroformate and dibutyl carbonate. The experimental conditions are shown in Table 6, and the experimental results are shown in Table 7.

**Table 6**

| Experiment number | Flow rate | | Photoreaction vessel volume | CH₄ total amount | Cl₂ total amount | Residence time | Reaction time |
|---|---|---|---|---|---|---|---|
| | Biogas (CH₄: mmol/min) | Air (O₂: mmol/min) | | | | | |
| 1 | 49.6mL/min (1.09mmol/min) | 75.0mL/min (0.66mmol/min) | 1652 mL | 98 mmol | 237 mmol | 10.3 min | 2.0h |
| 2 | 65.6mL/min (1.46mmol/min) | 99.3mL/min (0.88mmol/min) | 1544 mL | 99 mmol | | 7.2 min | 1. 6h |

**Table 7**

| Experiment number | Amount of produced carbonyl chloride | Carbonyl chloride conversion rate | Carbonyl chloride production efficiency |
|---|---|---|---|
| 1 | 87mmol | 89% | 65.4mmol/h |
| 2 | 94mmol | 95% | 83.2mmol/h |

Even when experiment was carried out on a larger scale, carbonyl chloride could be produced from biogas, chlorine gas and air with good yield as the result shown in Table 7.

### Example 5: Synthesis of carbonyl chloride using ozone in combination

The reaction system schematically shown in Figure 1 was used.

Calcium hypochlorite (39.0 g, 273 mmol) was added into a three-necked flask immersed in an ice bath. Chlorine gas was continuously produced by adding 2.1 mmol/L hydrochloric acid using a syringe pump at the injection rate of 1029 µL/min under atmospheric temperature while ultrasound of 70 W and 42 kHz was irradiated using the ultrasonic washer.

Ozone was produced using an ozone producing device ("R200" manufactured by WUOAUM). The amount of the produced ozone was 200 mg/h. The air containing ozone produced by the ozone producing device was dried by passing the air through a gas drying tube having the inner diameter of 16 mm and the length of 265 mm filled with silica gel. The flow rate of the dried air was adjusted to the flow rate shown in Table 8 using the mass flow controller, and the dried air was supplied into the above three-necked flask. The obtained mixed gas of chlorine and air was joined together with biogas ("TLF-06472" manufactured by AIR WATER) of which flow rate was adjusted using the mass flow controller to be supplied into the PTFE coil heater to prepare a mixed gas of chlorine/air/biogas. The mixed gas was supplied into the flow photoreactor. The used biogas was composed of methane: 53 vol%, carbon dioxide: 34 vol%, nitrogen: 8.3 vol% and oxygen: 2.5 vol%. The flow photoreactor was composed of a square borosilicate glass vessel (outer size: 220 mm × 185 mm × 50 mm, thickness: 3.3 mm, inner volume: 1652 cm³) and 365 nm LED lamp (manufactured by Polarstar, peak wavelength: 365 nm, 45 W, LED (14 cm × 18 cm), illumination intensity: 54.7 to 54.4 mW/cm²).

The temperatures of the flow photoreactor and the coil heater were not controlled during the reaction.

The gaseous product produced by the system was blown into 1-butanol in the first reaction vessel to produce the mixed product of chloroformate ester and carbonate.

The gas derived from the first reaction vessel was similarly supplied to the second reaction vessel containing 1-butanol. The reaction mixtures of the first reaction vessel and the second reaction vessel were analyzed by ¹H NMR, and the amount of the produced carbonyl chloride was determined by the amounts of the produced ethyl chloroformate and dibutyl carbonate. The experimental conditions and the experimental results are shown in Table 8.

**Table 8**

| Flow rate | | CH₄ total amount | Cl₂ total amount | Residence time | Reaction time | Carbonyl chloride | | |
|---|---|---|---|---|---|---|---|---|
| Biogas (CH₄: mmol/min) | Air (O₂: mmol/min) (O₃: mmol/min) | | | | | Amount produced | Conversion rate | Production efficiency |
| 65.6mL/min (1.45mmol/min) | 99.3mL/min (0.88mmol/min) (0.07mmol/min) | 99 mmol | 237 mmol | 6.6 min | 1. 6h | 91.5 mmol | 92% | 80.7 mmol/h |

Although ozone was used in combination with oxygen, the production efficiency of carbonyl chloride was not significantly improved.

### Example 6: Synthesis of carbonate

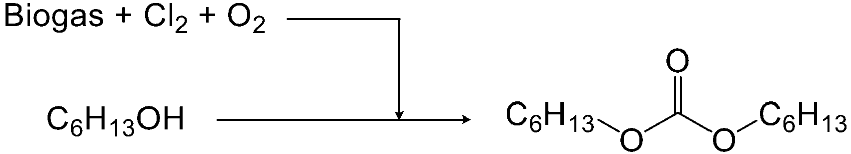

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to produce a photooxidation gas mainly containing carbonyl chloride and hydrogen chloride.

The produced photooxidation gas was blown into an acetonitrile solution (50 mL) containing 1-hexanol (30 mmol, 3.1 g) and pyridine (240 mmol, 19.2 mL), and the mixture was stirred at room temperature. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 55 minutes (CH₄ amount: 30 mmol) and the light of 365 nm was irradiated for 1 hour and 25 minutes, and air was supplied for 30 minutes to discharge the gas remaining in the reaction system. After the reaction, 1,2-dichloroethane (10 mmol, 0.8 mL) was added as an internal standard material to the reaction mixture, and the mixture was analyzed by ¹H NMR.

The production of dihexyl carbonate was confirmed in the solution after the reaction by ¹H NMR measurement (NMR yield: 99%) .

The obtained solution after the reaction was washed with 1 M hydrochloric acid and water, and extraction using CH₂Cl₂ was carried out. The obtained organic layer was dried over anhydrous sodium sulfate, and the solvent was removed by distillation under atmospheric pressure to obtain dihexyl carbonate as colorless liquid (amount: 3.2 g, 14.0 mmol, yield: 93%).

### Example 7: Synthesis of urea derivative

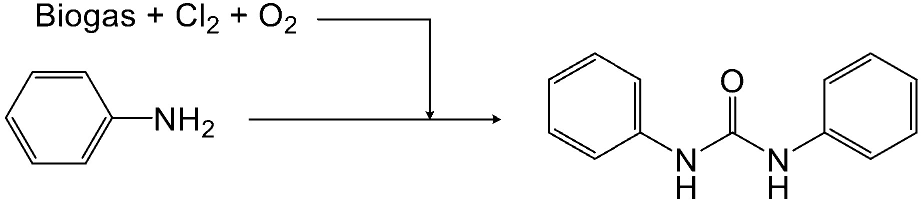

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to produce a photooxidation gas mainly containing carbonyl chloride and hydrogen chloride.

The produced photooxidation gas was blown into an acetonitrile solution (50 mL) containing aniline (30 mmol, 3.0 g) and pyridine (120 mmol, 9.6 mL), and the mixture was stirred at room temperature. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 55 minutes (CH₄ amount: 30 mmol) and the light of 365 nm was irradiated for 1 hour and 25 minutes, and air was supplied for 30 minutes to discharge the gas remaining in the reaction system. The solution was washed with 1 M hydrochloric acid and water after the reaction, and extraction using CH₂Cl₂ was carried out. The obtained organic layer was dried over anhydrous sodium sulfate, and the solvent was removed by distillation under reduced pressure. Diphenyl urea as colorless liquid (amount: 2.8 g, 13.0 mmol, yield: 88%) was obtained by the purification using a glass tube oven manufactured by Shibata Scientific.

### Example 8: Synthesis of N-protected amino acid

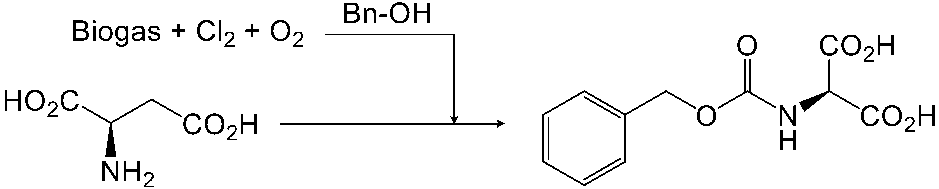

The reaction system schematically shown in Figure 1 was used.

Calcium hypochlorite (13.0 g, 91 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 40.0 mL/min (O₂ flow rate: 0.35 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W). Benzyl alcohol was injected through a T-shaped mixer connected to the entrance of the box-shaped photoreactor using a syringe pump at the flow rate of 25.8 µL/min for the reaction in a PTFE tube (inner diameter: 2.4 mm, length: 1000 mm, volume: 4.52 mL). The obtained product was added dropwise to a mixed solvent containing L-asparagine acid (5 mmol, 0.7 g), potassium carbonate (120 mmol, 23.8 g), water (30 mL) and acetonitrile (10 mL), and the mixture was stirred at room temperature for 18 hours.

The reaction system was stopped after the biogas was injected for 1 hour (CH₄ amount: 33.3 mmol) and the light of 365 nm was irradiated for 1 hour and 30 minutes, and air was supplied for 30 minutes to discharge the sample gas remaining in the reaction system. The gas discharged from the reaction system was treated to decompose the photooxidation product such as hydrogen chloride and excessive carbonyl chloride by supplying the gas into a sodium hydrogencarbonate aqueous solution.

After the reaction, 1,1,2,2-tetrachloroethane (5 mmol, 0.53 mL) was added as an internal standard material to the reaction mixture, and the mixture was analyzed by ¹H NMR. The production of N-Cbz-L-asparagine acid was confirmed in the solution after the reaction by ¹H NMR measurement (NMR yield: 20%).

Carbonyl chloride may be produced by reacting methane, chlorine and oxygen, and benzyl chloroformate may be produced by reacting the carbonyl chloride and benzyl alcohol to be further reacted with L-asparagine acid.

### Example 9: Synthesis of isocyanate compound

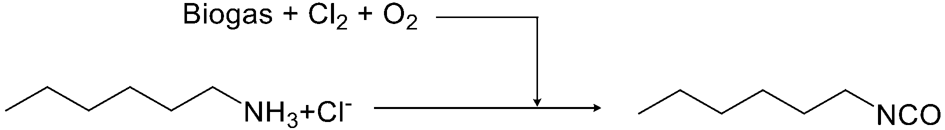

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into a toluene solution (50 mL) containing hexylamine hydrochloride (0.7 g, 5 mmol), and the mixture was stirred at 100°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 1 hour and 30 minutes (CH₄ amount: 50 mmol) and the light of 365 nm was irradiated for 2 hours, and air was supplied for 30 minutes to discharge the gas remaining in the reaction system. After the reaction, 1,1,2,2-tetrachloroethane (1.1 mL, 10 mmol) was added as an internal standard material to the reaction mixture, and the mixture was analyzed by ¹H NMR. The production of 1-isocyanatohexane was confirmed in the solution after the reaction by ¹H NMR measurement (NMR yield: 87%) .

### Example 10: Synthesis of isocyanate compound

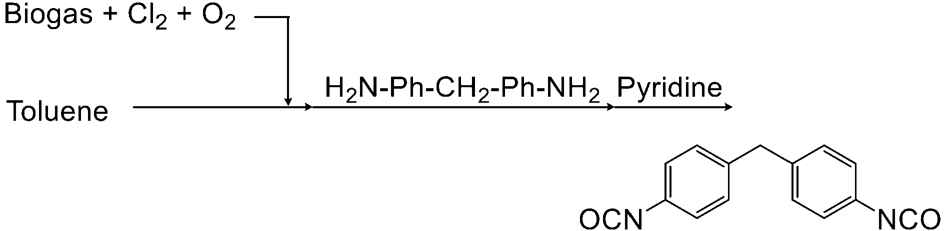

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 40.0 mL/min (O₂ flow rate: 0.35 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into a toluene (200 mL) in a three-necked round flask, and the mixture was stirred at 0°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 1 hour and 30 minutes (CH₄ amount: 50 mmol) and the light of 365 nm was irradiated for 2 hours. To the toluene into which the photooxidation gas was blown, the solution prepared by dissolving 4,4'-diaminodiphenylmethane (5 mmol, 1.0 g) in toluene (20mL) was injected. The mixture was stirred. Subsequently, the solution prepared by dissolving pyridine (50 mmol, 4.0 g) in toluene (10 mL) was injected, and the mixture was stirred. Then, the reaction vessel was heated to 60°C, and air was supplied thereto for 30 minutes to discharge the gas remaining in the reaction system.

After the reaction, 1,1,2,2-tetrachloroethane (1.1 mL, 10 mmol) was added as an internal standard material to the reaction mixture, and the mixture was analyzed by ¹H NMR. The production of methylenediphenyl 4,4'-diisocyanate was confirmed in the solution after the reaction by ¹H NMR measurement (NMR yield: 38%).

### Example 11: Vilsmeier reaction → esterification

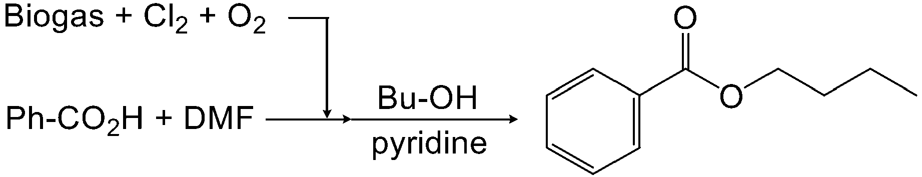

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into an acetonitrile solution (50 mL) containing benzoic acid (15 mmol, 1.8 g) and N,N-dimethylformamide (30 mmol, 2.3 mL), and the mixture was stirred at 30°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 55 minutes (CH₄ amount: 30 mmol) and the light of 365 nm was irradiated for 1 hour and 25 minutes, and air was supplied for 30 minutes to discharge the gas remaining in the reaction system. Then, 1-butanol (60 mmol, 5.5 mL) and pyridine (120 mmol, 9.6 mL) were added thereto, and the mixture was stirred for 1 hour at room temperature.

After the reaction, 1,2-dichloroethane (10 mmol, 0.8 mL) was added as an internal standard material to the reaction mixture, and the mixture was analyzed by ¹H NMR. The production of butyl benzoate was confirmed in the solution after the reaction by ¹H NMR measurement (NMR yield: >99%). The solvent was distilled away by the distillation at 90 to 150°C under atmospheric pressure. Then, butyl benzoate as colorless liquid was obtained by silica gel column chromatography (eluent: hexane/ethyl acetate = 5/1) (yield: 80%, amount: 2.1 g, 12 mmol).

### Example 12: Vilsmeier reaction → amidation

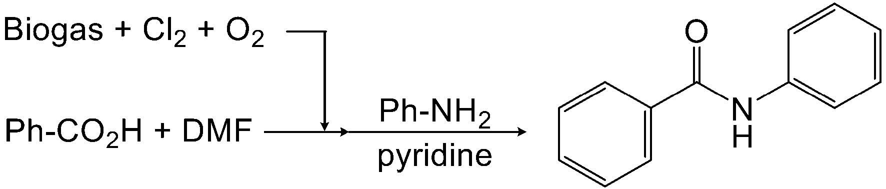

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into an acetonitrile solution (50 mL) containing benzoic acid (15 mmol, 1.8 g) and N,N-dimethylformamide (30 mmol, 2.3 mL), and the mixture was stirred at 30°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 55 minutes (CH₄ amount: 30 mmol) and the light of 365 nm was irradiated for 1 hour and 25 minutes, and air was supplied for 30 minutes to discharge the gas remaining in the reaction system. Then, aniline (60 mmol, 5.5 mL) and pyridine (120 mmol, 9.6 mL) were added thereto, and the mixture was stirred for 2 hours at room temperature.

After the reaction, 1,2-dichloroethane (10 mmol, 0.8 mL) was added as an internal standard material to the reaction mixture, and the mixture was analyzed by ¹H NMR. The production of benzanilide was confirmed in the solution after the reaction by ¹H NMR measurement (NMR yield: >99%). To the reaction mixture, 1 M hydrochloric acid and dichloromethane were added. The layers were separated. The organic layer was dried over anhydrous sodium sulfate, and the solvent was distilled away under reduced pressure to obtain benzanilide as red solid (yield: 84%, amount: 2.5 g, 13 mmol).

### Example 13: Formylation by Vilsmeier reaction

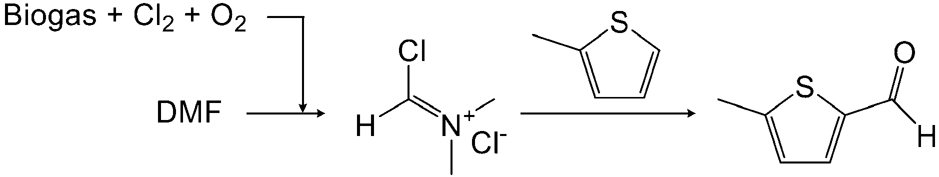

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into an acetonitrile solution (50 mL) containing N,N-dimethylformamide (50 mmol, 3.8 mL), and the mixture was stirred at 30°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 1 hour and 30 minutes (CH₄ amount: 50 mmol) and the light of 365 nm was irradiated for 2 hours, and air was supplied for 30 minutes to discharge the gas remaining in the reaction system. Next, 2-methylthiophene (10 mmol, 1.9 g) was added thereto at 0°C, and then the mixture was stirred at 70°C for 30 minutes. Then, a sodium carbonate aqueous solution (80 mL, 240 mmol) was added to the reaction mixture, and the mixture was stirred at room temperature for 30 minutes.

Water and dichloromethane were added to the reaction mixture after the reaction, and the layers were separated. The organic layer was dried over anhydrous sodium sulfate, and the solvent was distilled away under reduced pressure to obtain 5-methylthiophene-2-carboxaldehyde as yellow liquid (yield: 85%, amount: 1.1 g, 8.5 mmol).

### Example 14: Synthesis of carbamoyl chloride

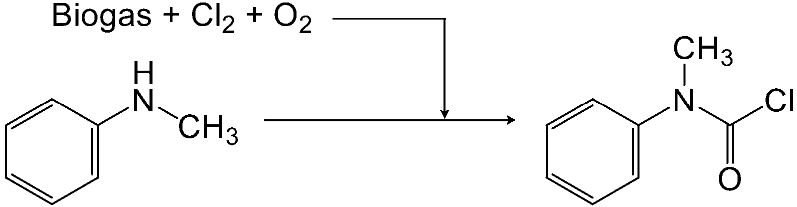

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into an acetonitrile solution (50 mL) containing N-methylaniline (3 mmol, 0.32 g) and N,N-diisopropylethylamine (80 mmol, 14 mL), and the mixture was stirred at atmospheric temperature. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 55 minutes (CH₄ amount: 30 mmol) and the light of 365 nm was irradiated for 1 hour and 25 minutes, and air was supplied for 30 minutes to discharge the gas remaining in the reaction system.

After the reaction, 1,2-dichloroethane (10 mmol, 0.8 mL) was added as an internal standard material to the reaction mixture, and the mixture was analyzed by ¹H NMR. The production of N-methyl-N-phenylcarbamyl chloride was confirmed in the solution after the reaction by ¹H NMR measurement (NMR yield: >99%).

The obtained reaction mixture was washed with an aqueous solvent, and the obtained organic layer was dried over anhydrous sodium sulfate, and the solvent was distilled away under atmospheric pressure. The obtained residue was subjected to silica gel column chromatography (eluent: ethyl acetate/hexane = 5/1) and recrystallization using dichloromethane/hexane to obtain N-methyl-N-phenylcarbamyl chloride as a white solid (yield: 88%, amount: 0.45 g, 2.7 mmol).

### Example 15: Synthesis of α-amino acid-N-carboxylic anhydride (NCA)

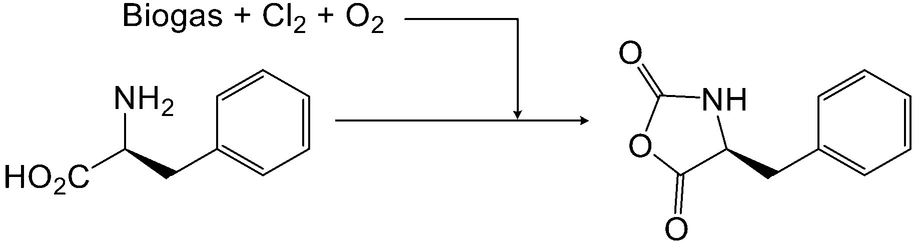

The reaction system schematically shown in Figure 1 was used.

Calcium hypochlorite (11.9 g, 83 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into an acetonitrile solution (30 mL) containing L-phenylalanine (1 mmol, 0.17 g), and the mixture was stirred at 70°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 3 hours and 18 minutes (CH₄ amount: 110 mmol) and the light of 365 nm was irradiated for 3 hours and 48 minutes, and air was supplied for 30 minutes to discharge the gas remaining in the reaction system.

After the reaction, 1,2-dichloroethane (10 mmol, 0.8 mL) was added as an internal standard material to the reaction mixture, and the mixture was analyzed by ¹H NMR. The production of L-phenylalanine-NCA was confirmed in the solution after the reaction by ¹H NMR measurement (NMR yield: >99%). To the reaction mixture, 1 M hydrochloric acid and dichloromethane were added. The layers were separated. The organic layer was dried over anhydrous sodium sulfate, and the solvent was distilled away under atmospheric pressure. Recrystallization using dichloromethane/hexane was conducted to obtain L-phenylalanine-NCA as a yellow solid (yield: >99%, amount: 0.19 g, 1 mmol).

### Example 16: Synthesis of polycarbonate

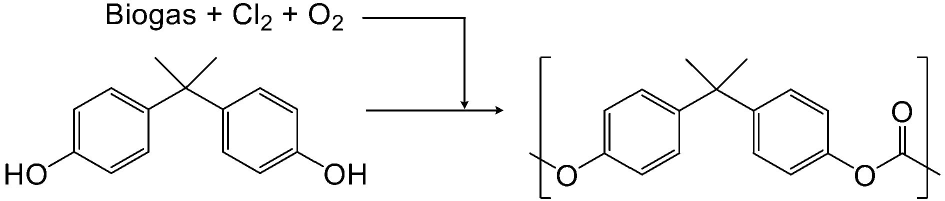

The reaction system schematically shown in Figure 1 was used.

Calcium hypochlorite (15.6 g, 109 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel was supplied thereto using the mass flow controller at the flow rate of 55.5 mL/min (O₂ flow rate: 0.49 mmol/min) to prepare a mixed gas of chlorine and air. The produced mixed gas was further mixed with biogas of which flow rate was 24.8 mL/min (CH₄ flow rate: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W) to obtain a photooxidation gas mainly containing carbonyl chloride and hydrogen chloride.

The produced photooxidation gas was blown into an acetonitrile solution (50 mL) containing Bisphenol A (10 mmol, 2.28 g) and pyridine (200 mmol, 16.1 mL), and the mixture was stirred at 30°C. The gas discharged from the reaction system was treated to decompose the photooxidation product such as hydrogen chloride and excessive carbonyl chloride by supplying the gas into a sodium hydrogencarbonate aqueous solution.

The reaction system was stopped after the biogas was injected for 1 hour and 12 minutes (CH₄ amount: 40 mmol) and the light of 365 nm was irradiated for 1 hour and 42 minutes, and air was supplied at 50°C for 1 hour to discharge the gas remaining in the reaction system.

Methanol was added to the reaction mixture after the reaction to precipitate the polymer. The polymer was filtrated to obtain polycarbonate as a white solid (yield: 88%, amount: 2.24 g, 8.8 mmol).

The produced polycarbonate Bisphenol A was analyzed by gel permeation chromatography (GPC) to determine the molecular weight. The result is shown in Table 9.

**Table 9**

| Mw | Mn | Mw/Mn |
|---|---|---|
| 123,700 | 88,900 | 1.39 |

### Example 17: Synthesis of carboxylic acid chloride

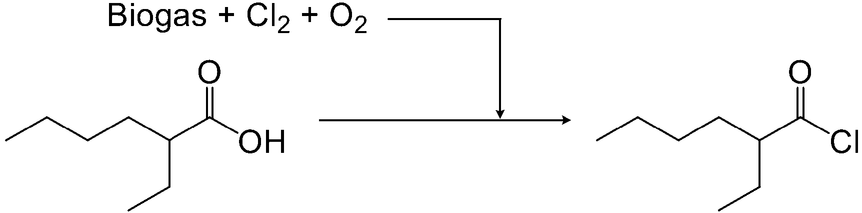

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel (40.0 mL/min, O₂: 0.35 mmol/min) was supplied thereto using the mass flow controller to prepare a mixed gas of chlorine and air. The produced mixed gas was mixed with biogas (24.8 mL/min, CH₄: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W). The produced photooxidation gas was blown into a toluene solution (3.0 mL) containing 2-ethylhexanoic acid (40.5 mmol, 5.8 g) and DMF (40.5 mmol, 3.1 mL), and the mixture was stirred at room temperature. The discharged gas was blown into a sodium hydrogencarbonate aqueous solution to decompose the photooxidation product such as hydrogen chloride and excessive carbonyl chloride. Biogas was injected for 1 hour and 30 minutes (CH₄: 50 mmol) and the light of 365 nm was irradiated for 2 hours, and then air was supplied for 30 minutes to discharge the gas remaining in the reaction system.

After the reaction, 1,1,2,2-tetrachloroethane (10 mmol, 1.1 mL) was added as an internal standard material to the reaction mixture, and the mixture was analyzed by ¹H NMR. As a result, it was confirmed that 2-ethylhexanoyl chloride was produced as the target compound with the yield of 60% (24.3 mmol) to the used carboxylic acid. Carbonyl chloride might be produced from the methane contained in the biogas by oxidative photoreaction, the carbonyl chloride might be reacted with DMF to produce a Vilsmeier reagent, and 2-ethylhexanoic acid might be converted into the chloride thereof due to the Vilsmeier reagent.

### Example 18: Synthesis of Vilsmeier reagent and dehydration reaction thereby

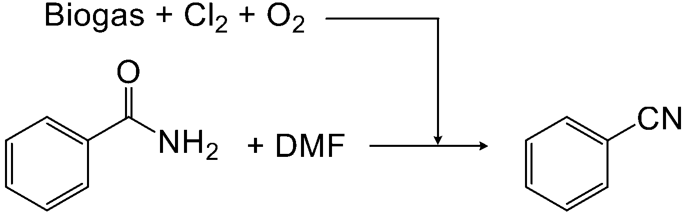

The reaction system schematically shown in Figure 1 was used.

Calcium hypochlorite (7.2 g, 50 mmol) was added into a three-necked round flask, and the flask was immersed in an ultrasonic generator. Chlorine gas was continuously produced by adding 35% hydrochloric acid using a syringe pump at the flow rate of 429 µL/min while ultrasound was irradiated. Air dewatered by silica gel (38.5 mL/min, O₂: 0.34 mmol/min) was supplied thereto using the mass flow controller to prepare a mixed gas of chlorine and air. The produced mixed gas was mixed with biogas (24.8 mL/min, CH₄: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W). The produced photooxidation gas was blown into a chloroform solution (30 mL) containing benzamide (5 mmol, 0.61 g) and dimethylformamide (25 mmol, 1.83 g), and the mixture was stirred at room temperature. The discharged gas was blown into a sodium hydrogencarbonate aqueous solution to decompose the photooxidation product such as hydrogen chloride and excessive carbonyl chloride. Biogas was injected for 30 minutes (CH₄: 0.55 mmol) and the light of 365 nm was irradiated for 1 hour, and then air was supplied for 30 minutes to discharge the gas remaining in the reaction system.

After the reaction, 1,1,2,2-tetrachloroethane (5 mmol, 0.53 mL) was added as an internal standard material to the reaction mixture, and the mixture was analyzed by ¹H NMR. As a result, it was confirmed that benzonitrile was produced as the target compound with the yield of >99% to the used amide.

Carbonyl chloride produced by the photoreaction might be reacted with DMF to produce a Vilsmeier reagent, and the Vilsmeier reagent might contribute to the dehydration reaction of the carbamoyl group of benzamide.

### Example 19: Synthesis of carbonate

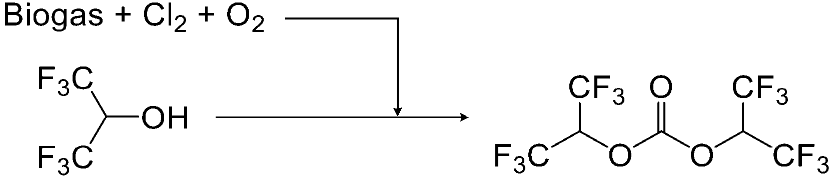

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel (40.0 mL/min, O₂: 0.35 mmol/min) was supplied thereto using the mass flow controller to prepare a mixed gas of chlorine and air. The produced mixed gas was mixed with biogas (24.8 mL/min, CH₄: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W). The produced photooxidation gas was blown into a dichloromethane solution (10 mL) containing hexafluoro-2-propanol (40 mmol, 4.62 mL) and pyridine (160 mmol, 12.8 mL), and the mixture was stirred at room temperature. The discharged gas was blown into a sodium hydrogencarbonate aqueous solution to decompose the photooxidation product such as hydrogen chloride and excessive carbonyl chloride. Biogas was injected for 1 hour and 30 minutes (CH₄: 50 mmol) and the light of 365 nm was irradiated for 2 hours, and then air was supplied for 30 minutes to discharge the gas remaining in the reaction system.

After the reaction, 1,1,2,2-tetrachloroethane (10 mmol, 1.1 mL) was added as an internal standard material to the reaction mixture, and the mixture was analyzed by ¹H NMR. As a result, it was confirmed that bis(1,1,1,3,3,3-hexafluoropropane-2-yl)carbonate was produced as the target compound with the yield of 98% (19.6 mmol) to the used alcohol.

### Example 20: Synthesis of isocyanate

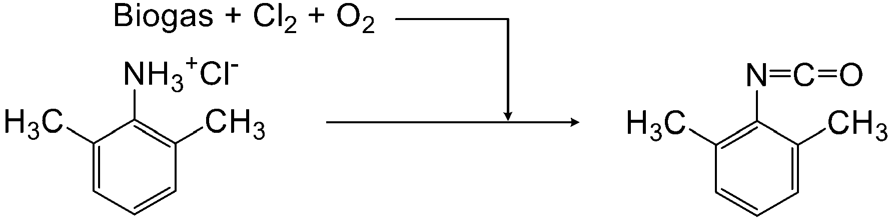

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel (40.0 mL/min, O₂: 0.35 mmol/min) was supplied thereto using the mass flow controller to prepare a mixed gas of chlorine and air. The produced mixed gas was mixed with biogas (24.8 mL/min, CH₄: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W). The produced photooxidation gas was blown into a toluene suspension (50 mL) containing 2,6-dimethylaniline hydrochloride (5 mmol, 0.8 g), and the mixture was stirred at 100°C. The discharged gas was blown into a sodium hydrogencarbonate aqueous solution to decompose the photooxidation product such as hydrogen chloride and excessive carbonyl chloride. Biogas was injected for 1 hour and 30 minutes (CH₄: 50 mmol) and the light of 365 nm was irradiated for 2 hours, and then air was supplied for 30 minutes to discharge the gas remaining in the reaction system.

After the reaction, 1,1,2,2-tetrachloroethane (5 mmol, 0.5 mL) was added as an internal standard material to the reaction mixture, and the mixture was analyzed by ¹H NMR. As a result, it was confirmed that 2,6-dimethylphenylisocyanate was produced as the target compound with the yield of 34% (1.7 mmol) to the used 2,6-dimethylaniline hydrochloride.

### Example 21: Synthesis of isocyanate

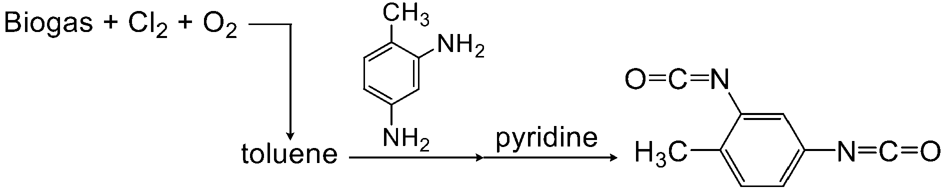

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel (40.0 mL/min, O₂: 0.35 mmol/min) was supplied thereto using the mass flow controller to prepare a mixed gas of chlorine and air. The produced mixed gas was mixed with biogas (24.8 mL/min, CH₄: 0.55 mmol/min).

The produced mixed gas was supplied into a box-shaped photoreactor of 220 mm × 185 mm × 30 mm (volume: 891 mL), and light was irradiated thereto using 365 nm LED lamp (45 W). The produced photooxidation gas was blown into toluene (200 mL), and the mixture was stirred at 0°C. The discharged gas was blown into a sodium hydrogencarbonate aqueous solution to decompose the photooxidation product such as hydrogen chloride and excessive carbonyl chloride. Biogas was injected for 1 hour and 30 minutes (CH₄: 50 mmol) and the light of 365 nm was irradiated for 2 hours, and then a solution prepared by 2,4-diaminotoluene (7 mmol, 0.9 g) in toluene (50 mL) was injected in the above-described three-necked round flask containing the above-described toluene, and the mixture was stirred. Subsequently, a solution prepared by dissolving pyridine (50 mmol, 4.0 g) in toluene (10 mL) was injected thereto, and the mixture was stirred. Then, the vessel was heated to 70°C and air was supplied for 30 minutes to discharge the gas remaining in the reaction system.

Next, the reaction mixture was washed with an aqueous solvent, and the obtained organic layer was dried over anhydrous sodium sulfate, and the solvent was distilled away under reduced pressure. To the obtained residue, 1,2-dichloroethane (5 mmol, 0.4 mL) was added as an internal standard material. The mixture was analyzed by ¹H NMR. As a result, it was confirmed that tolylene-2,4-diisocyanate was produced as the target compound with the yield of 39% (2.0 mmol) to the used 2,4-diaminotoluene.

### Example 22: Synthesis of carbonyl chloride from digester gas

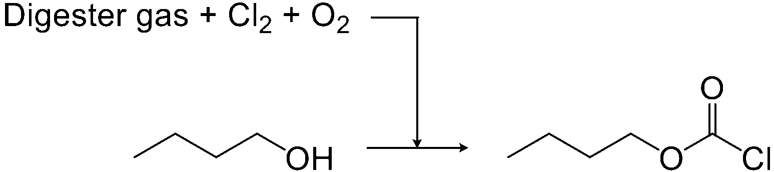

The reaction system schematically shown in Figure 1 was used.

Calcium hypochlorite (19.5 g, 136 mmol) was added into a three-necked flask immersed, and the flask was immersed in an ice bath. Chlorine gas was continuously produced by adding 2.1 mmol/L hydrochloric acid using a syringe pump at atmospheric temperature at the flow rate of 389 µL/min while ultrasound of 70 W and 42 kHz was irradiated by the ultrasonic washer.

Air to be dried was sent to a gas drying tube having the inner diameter of 16 mm and the length of 265 mm filled with silica gel using the air pump and the mass flow controller, and sent to the above three-necked flask at the flow rate of 32 mL (O₂ flow rate: 0.28 mmol/min). The obtained mixed gas of chlorine and air was joined together with digester gas of which flow rate was adjusted to 21.9 mL/min (methane flow rate: 0.55 mmol/min) using the mass flow controller to be supplied into the PTFE coil heater to prepare a mixed gas of chlorine/air/digester gas. The mixed gas was supplied into the flow photoreactor. The digester gas was provided from the sewage plant of a certain city and composed of methane: 59.2 vol%, carbon dioxide: 39.6 vol%, nitrogen: 0.3 vol%, oxygen: less than 0.2 vol%, moisture content: 1.2 vol%, other gas: less than 0.1 vol% and hydrogen sulfide: less than 0.01 ppm by volume. The flow photoreactor was composed of a square borosilicate glass vessel of 220 mm × 185 mm × 30 mm having the thickness of 3.3 mm and the inner volume of 891 cm³ and LED lamp (manufactured by Polarstar, peak wavelength: 365 nm, 45 W, LED size: 14 cm × 18 cm, illumination intensity: 54.7 to 54.4 mW/cm²).

The residence time of the reaction gas in the flow photoreactor was 10.4 minutes. The temperatures of the flow photoreactor and the coil heater were not controlled during the reaction.

The gas derived from the flow photoreactor was blown into 1-butanol in the first reaction vessel, and the gas derived from the first reaction vessel was similarly supplied to the second reaction vessel containing 1-butanol. After the digester gas was injected for 1 hour and 30 minutes (CH₄ amount: 49 mmol) and the light of 365 nm was irradiated for 2 hours, air was supplied for 30 minutes to discharge the gas remaining in the reaction system. The reaction mixtures in the first reaction vessel and the second reaction vessel were analyzed by ¹H NMR, and the amount of the produced carbonyl chloride was determined by the amounts of the produced ethyl chloroformate and dibutyl carbonate. The result is shown in Table 10.

**Table 10**

| A: chloroformate ester | B: carbonate | Carbonyl chloride amount (A + B) | Carbonyl chloride conversion rate |
|---|---|---|---|
| 45mmol | 0mmol | 45mmol | 92% |

It was found that carbonyl chloride can be successfully produced from the biogas provided from a sewage plant by the present invention method as the result shown in Table 10.

### Example 23: Synthesis of isocyanate from digester gas

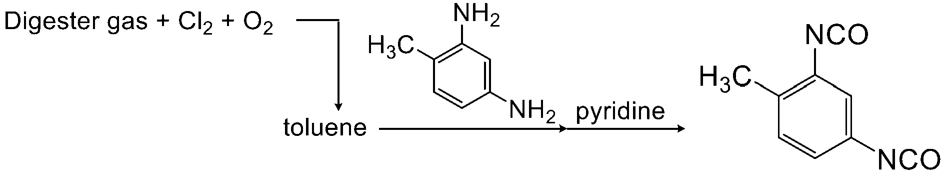

The reaction system schematically shown in Figure 1 was used.

Calcium hypochlorite (19.5 g, 136 mmol) was added into a three-necked round flask, and the flask was immersed in an ultrasonic generator. Chlorine gas was continuously produced by adding 35% hydrochloric acid using a syringe pump at the flow rate of 388.9 µL/min while ultrasound was irradiated. Air dewatered by silica gel (32.0 mL/min, O₂: 0.28 mmol/min) was supplied thereto using the mass flow controller to prepare a mixed gas of chlorine and air. The mixed gas was mixed with a digester gas (21.9 mL/min, CH₄: 0.54 mmol/min). The digester gas was provided from the sewage plant of a certain city and composed of methane: 59.2 vol%, carbon dioxide: 39.6 vol%, nitrogen: 0.3 vol%, oxygen: less than 0.2 vol%, moisture content: 1.2 vol%, other gas: less than 0.1 vol% and hydrogen sulfide: less than 0.01 ppm by volume.

The obtained mixed gas was supplied into a box-shaped photoreactor of 220 mm × 185 mm × 30 mm (volume: 891 mL), and light was irradiated thereto using 365 nm LED lamp (45 W). The produced photooxidation gas was blown into toluene (200 mL) in a three-necked round flask, and the mixture was stirred at 0°C. The discharged gas was blown into a sodium hydrogencarbonate aqueous solution to decompose the photooxidation product such as hydrogen chloride and excessive carbonyl chloride. Biogas was injected for 1 hour and 30 minutes (CH₄: 49 mmol) and the light of 365 nm was irradiated for 2 hours, and then a solution prepared by dissolving 2,4-diaminotoluene (5 mmol, 0.6 g) in toluene (50 mL) was injected in the above-described three-necked round flask containing toluene, and the mixture was stirred. Subsequently, a solution prepared by dissolving pyridine (50 mmol, 4.0 g) in toluene (10 mL) was injected thereto, and the mixture was stirred. Then, the vessel was heated to 70°C and air was supplied for 30 minutes to discharge the gas remaining in the reaction system.

Then, the reaction mixture was washed with an aqueous solvent, and the obtained organic layer was dried over anhydrous sodium sulfate, and the solvent was distilled away under reduced pressure. To the obtained residue, 1,2-dichloroethane (5 mmol, 0.4 mL) was added as an internal standard material. The mixture was analyzed by ¹H NMR. As a result, it was confirmed that tolylene-2,4-diisocyanate was produced as the target compound with the yield of 43% (2.2 mmol, 0.37 g) to the used 2,4-diaminotoluene.

### DESCRIPTION OF REFERENCE NUMERALS

1: Silica gel drying tube, 2: Mass flow controller
3: Halogen molecule gas generator, 4: Calcium chloride drying tube
5: Methane gas cylinder, 6: Coil heater, 7: Photoreaction vessel
8: Heater, 9: Light source, 10: Reaction vessel

## Claims

1. A method for producing a carbonyl halide, the method comprising the step of:
irradiating a light to a mixed gas comprising methane, a halogen molecule gas and oxygen.

2. The method according to claim 1, wherein the carbonyl halide is carbonyl chloride, and the halogen molecule gas is a chlorine gas.

3. The method according to claim 1, wherein the light has a peak wavelength of 360 nm or more and 830 nm or less.

4. The method according to claim 1, wherein the light is irradiated to the mixed gas at an atmospheric temperature.

5. The method according to claim 1, wherein the shortest distance between a light source to irradiate the light and the mixed gas is 1 m or less.

6. The method according to claim 1, further comprising the step of producing a sodium halide aqueous solution by neutralizing a hydrogen halide with a basic sodium salt, wherein the hydrogen halide is produced as a side product by irradiating the light to the mixed gas.

7. The method according to claim 1, further comprising the step of electrolyzing the sodium halide aqueous solution to produce the halogen molecule and sodium hydroxide.

8. A method for producing a carbonate compound, the method comprising the steps of:
producing a carbonyl halide by the method according to any one of claims 1 to 7, and
reacting an alcohol compound and the carbonyl halide.

9. A method for producing a halogenated formate ester compound, the method comprising the steps of:
producing a carbonyl halide by the method according to any one of claims 1 to 7, and
reacting an alcohol compound and the carbonyl halide.

10. A method for producing an isocyanate compound, the method comprising the steps of:
producing a carbonyl halide by the method according to any one of claims 1 to 7, and
reacting a primary amine compound and the carbonyl halide.

11. A method for producing a carbamoyl halide compound, the method comprising the steps of:
producing a carbonyl halide by the method according to any one of claims 1 to 7, and
reacting a secondary amine compound and the carbonyl halide.

12. A method for producing an amino acid-N-carboxylic anhydride, the method comprising the steps of:
producing a carbonyl halide by the method according to any one of claims 1 to 7, and
reacting an amino acid compound represented by the following formula (VII) and the carbonyl halide,
wherein the amino acid-N-carboxylic anhydride is represented by the following formula (VIII): 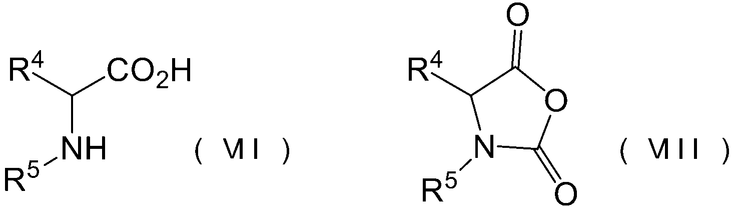 wherein
R⁴ is an amino acid side chain group wherein a reactive group is protected,
R⁵ is H or P¹-[-NH-CHR⁶-C(=O)-]ₗ- wherein R⁶ is an amino acid side chain group wherein a reactive group is protected, P¹ is a protective group of the amino group, 1 is an integer of 1 or more, and when 1 is an integer of 2 or more, a plurality of R⁶ may be the same as or different from each other.

13. A method for producing a Vilsmeier reagent,
wherein the Vilsmeier reagent is a salt represented by the following formula (X): 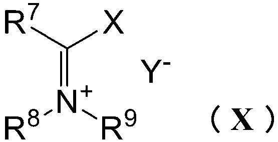 wherein
R⁷ is a hydrogen atom, a C₁₋₆ alkyl group or an optionally substituted C₆₋₁₂ aromatic hydrocarbon group,
R⁸ and R⁹ are independently a C₁₋₆ alkyl group or an optionally substituted C₆₋₁₂ aromatic hydrocarbon group, or R⁸ and R⁹ may form a 4 or more and 7 or less-membered ring structure together with each other,
X is a halogeno group selected from the group consisting of chloro, bromo and iodo,
Y⁻ is a counter anion,
the method comprising the steps of:
producing a carbonyl halide by the method according to any one of claims 1 to 7, and
reacting the carbonyl halide and an amide compound represented by the following formula (IX): 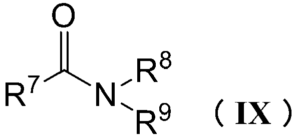
wherein R⁷ to R⁹ have the same meanings as the above.

14. A method for producing a urea compound, the method comprising the steps of:
producing a carbonyl halide by the method according to any one of claims 1 to 7, and
reacting a primary amine compound or a secondary amine compound and the carbonyl halide.
